(19) Europäisches Patentamt / European Patent Office / Office européen des brevets

(11) **EP 4 736 737 A1**

(12) # EUROPEAN PATENT APPLICATION
published in accordance with Art. 153(4) EPC

(43) Date of publication:
**06.05.2026 Bulletin 2026/19**

(21) Application number: **24832019.4**

(22) Date of filing: **26.06.2024**

(51) International Patent Classification (IPC):
**A61B 1/06** (2006.01)        **A61B 1/00** (2006.01)

(52) Cooperative Patent Classification (CPC):
**A61B 1/00; A61B 1/06**

(86) International application number:
**PCT/JP2024/023262**

(87) International publication number:
**WO 2025/005160 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.06.2023 JP 2023108688**

(71) Applicant: **Sony Olympus Medical Solutions Inc.
Hachioji-shi, Tokyo 192-0904 (JP)**

(72) Inventor: **KOJIMA, Koji
Hachioji-shi, Tokyo 192-0904 (JP)**

(74) Representative: **D Young & Co LLP
3 Noble Street
London EC2V 7BQ (GB)**

(54) **MEDICAL CONTROL DEVICE AND MEDICAL OBSERVATION SYSTEM**

(57)   A medical control device 9 includes: a light source controller 941 that executes a first control of emitting first light from a light source device 3 and a second control of emitting excitation light from the light source device 3; an imaging controller 942 that controls operations of a first image sensor 531 configured to image light including at least a part of a visible light wavelength band in return light from the observation target OB and operations of a second image sensor 532 configured to image light including at least a part of a wavelength band of observation target fluorescence in the return light; and a mode switcher 943. The light source controller 941 sets a control value in the first control to a control value being a control threshold or less in a first mode, and sets the control value in the first control to a control value larger than the control threshold in a second mode. The control threshold is a maximum value of the control value in which a signal value based on observation target fluorescence is larger than a signal value based on unnecessary light imaged by the second image sensor 532 and generated at least due to the first light.

FIG.11

**Description**

Field

The present disclosure relates to a medical control device and a medical observation system. Background

Conventionally, there has been known a medical observation system (refer to Patent Literature 1, for example) that irradiates an observation target (subject such as a person) with first light including visible light such as excitation light being narrowband light or white light being broadband light emitted from a light source device, and observes, by the application of the excitation light, fluorescence emitted from a substance contained in the observation target (hereinafter, denoted as observation target fluorescence).

With such fluorescence observation, tissue conditions that are difficult to recognize can be grasped through observation target fluorescence. Therefore, fluorescence observation can be utilized for various purposes and applications such as identification of a lesion.

Citation List

Patent Literature

Patent Literature 1: JP 2021-132695 A

Summary

Technical Problem

Meanwhile, the medical observation system has an observation optical path including: a first optical path used for propagation of first light from the light source device to the observation target; and a second optical path used for propagation of second light being return light (including observation target fluorescence) of the first light, returned from the observation target irradiated with the first light. When the first and second light propagate through the observation optical path, optical elements constituting the observation optical path are irradiated with the first and second light, thereby generating autofluorescence from the optical elements. Such autofluorescence has a wavelength band including a wavelength band of observation target fluorescence (which is a target of fluorescence observation), and will be noise in performing the fluorescence observation.

This leads to a demand for a technique capable of satisfactorily performing fluorescence observation.

The present disclosure has been made in view of the above, and aims to provide a medical control device and a medical observation system capable of performing fluorescence observation satisfactorily.

Solution to Problem

To solve the above-described problem and achieve the object, a medical control device according to the present disclosure includes: a light source controller configured to perform: a first control of emitting first light including at least a part of a visible light wavelength band from a light source device; and a second control of emitting excitation light that excites a substance contained in an observation target from the light source device; an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; and a mode switcher configured to switch a mode to a first mode or a second mode, wherein the light source controller is configured to set a control value in the first control to a control value of a control threshold or less in the first mode, and set the control value in the first control to a control value larger than the control threshold in the second mode, and the control threshold is a maximum value of the control value in which a signal value based on the observation target fluorescence is larger than a signal value based on unnecessary light imaged by the second image sensor and generated at least due to the first light.

Moreover, a medical control device according to the present disclosure includes: a light source controller configured to perform: a first control of emitting first light including at least a part of a visible light wavelength band from a light source device; and a second control of emitting excitation light that excites a substance contained in an observation target from the light source device; an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; a mode switcher configured to switch a mode to a first mode or a second mode; and a brightness controller configured to control brightness of an image based on a pixel signal output from the first image sensor, wherein the light source controller is configured to set a control value in the first control to a control value of a control threshold or less in the first mode, and set the control value in the first control to a control value larger than the control threshold in the second mode, and the brightness controller is configured to control the brightness such that the brightness of the image based on the pixel signal output from the first image sensor in the first mode is adjusted to be closer to the brightness of the image in the second mode.

Moreover, a medical control device according to the present disclosure includes: a light source controller configured to perform: a first control of emitting first light including at least a part of a visible light wavelength band from a light source device; and a second control of emitting excitation light that excites a substance contained in an observation target from the light source device; an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; a mode switcher configured to switch to a first mode or a second mode; and a brightness controller configured to adjust brightness of an image based on a pixel signal output from the first image sensor, wherein the light source controller is configured to set a control value in the first control to a control value of a control threshold or less in the first mode, and set the control value in the first control to a control value larger than the control threshold in the second mode, the brightness controller is configured to set a parameter in the first mode to a larger value than the parameter in the second mode, and the parameter is at least one of: an exposure time of the first image sensor; an analog gain to adjust the brightness of the image based on the pixel signal output from the first image sensor; or a digital gain to adjust the brightness of the image based on the pixel signal output from the first image sensor.

Moreover, a medical observation system according to the present disclosure includes: a light source device configured to emit each light of first light including at least a part of a visible light wavelength band and excitation light that excites a substance contained in an observation target; an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and a medical control device configured to control individual operations of the light source device and the imaging device, wherein the medical control device includes: a light source controller configured to execute: a first control of emitting the first light from the light source device; and a second control of emitting the excitation light from the light source device; an imaging controller configured to control operations of the imaging device; and a mode switcher is configured to switch a mode to a first mode or a second mode, the light source controller is configured to set a control value in the first control to a control value of a control threshold or less in the first mode, and set the control value in the first control to a control value larger than the control threshold in the second mode, and the control threshold is a maximum value of the control value in which a signal value based on the observation target fluorescence is larger than a signal value based on unnecessary light imaged by the second image sensor and generated at least due to the first light.

Moreover, a medical observation system according to the present disclosure includes: a light source device configured to emit each light of first light including at least a part of a visible light wavelength band and excitation light that excites a substance contained in an observation target; an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and a medical control device configured to control individual operations of the light source device and the imaging device, wherein the medical control device includes: a light source controller configured to execute: a first control of emitting the first light from the light source device; and a second control of emitting the excitation light from the light source device; an imaging controller configured to control operations of the imaging device; a mode switcher configured to switch a mode to a first mode or a second mode; and a brightness controller configured to control brightness of an image based on a pixel signal output from the first image sensor, the light source controller is configured to set a control value in the first control in the first mode to a control value of a control threshold or less, and set the control value in the first control in the second mode to a control value larger than the control threshold, and the brightness controller is configured to control the brightness such that the brightness of the image based on the pixel signal output from the first image sensor in the first mode is adjusted to be closer to the brightness of the image in the second mode.

Moreover, a medical observation system according to the present disclosure includes: a light source device configured to emit each light of first light including at least a part of a visible light wavelength band and excitation light that excites a substance contained in an observation target; an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and a medical control device configured to control individual operations of the light source device and the imaging device, wherein the medical control device includes: a light source controller configured to execute: a first control of emitting the first light from the light source device; and a second control of emitting the excitation light from the light source device; an imaging controller configured to control operations of the imaging device; a mode switcher configured to switch a mode to a first mode or a second mode; and a brightness controller that controls brightness of an image based on a pixel signal output from the first image sensor, the light source controller is configured to set a control value in the first control in the first mode to a control value of a control threshold or less, and set the control value in the first control in the second mode to a control value larger than the control threshold, and the brightness controller is configured to set a parameter in the first mode to a value larger than the parameter in the second mode, and the parameter is at least one of: an exposure time of the first image sensor; an analog gain to adjust the brightness of the image based on the pixel signal output from the first image sensor; or a digital gain to adjust the brightness of the image based on the pixel signal output from the first image sensor. Advantageous Effects of Invention

With the medical control device and the medical observation system according to the present disclosure, fluorescence observation can be performed satisfactorily.

Brief Description of Drawings

FIG. 1 is a diagram illustrating a configuration of a medical observation system according to a first embodiment.
FIG. 2 is a block diagram illustrating a configuration of a camera head and a control device.
FIG. 3 is a diagram illustrating functions of a light source controller and an imaging controller.
FIG. 4 is a diagram illustrating functions of the light source controller and the imaging controller.
FIG. 5 is a diagram illustrating functions of a light source controller and an imaging controller.
FIG. 6 is a diagram illustrating functions of a light source controller and an imaging controller.
FIG. 7 is a diagram illustrating a method of setting a lighting period of a first light source in third light source control.
FIG. 8 is a diagram illustrating a method of setting the lighting period of the first light source in the third light source control.
FIG. 9 is a diagram illustrating a method of setting the lighting period of the first light source in the third light source control.
FIG. 10 is a diagram illustrating a method of setting the lighting period of the first light source in the third light source control.
FIG. 11 is a diagram illustrating an S/N ratio calculation method.
FIG. 12 is a diagram illustrating a configuration of a medical observation system according to a second embodiment.
FIG. 13 is a diagram illustrating modification 2-1 of the second embodiment.
FIG. 14 is a diagram illustrating modification 2-2 of the second embodiment.
FIG. 15 is a diagram illustrating modification 2-7 of the second embodiment.
FIG. 16 is a diagram illustrating modification 2-7 of the second embodiment.
FIG. 17 is a diagram illustrating modification 2-9 of the second embodiment.
FIG. 18 is a diagram illustrating modification 3-1 of the first and second embodiments.
FIG. 19 is a diagram illustrating modification 3-2 of the first and second embodiments.
FIG. 20 is a diagram illustrating modification 3-3 of the first and second embodiments.
FIG. 21 is a diagram illustrating modification 3-3 of the first and second embodiments.

Description of Embodiments

Hereinafter, modes for carrying out the present disclosure (hereinafter referred to as embodiments) will be described with reference to the drawings. Note that the present disclosure is not limited to the embodiments described below. In the drawings, same reference signs are attached to the same components.

(First embodiment)

[Configuration of medical observation system]

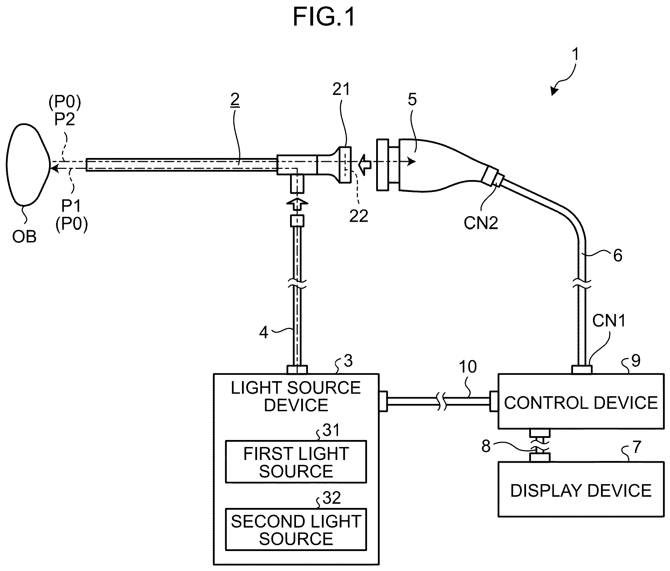

FIG. 1 is a diagram illustrating a configuration of a medical observation system 1 according to a first embodiment.

In the first embodiment, the medical observation system 1 is a medical endoscope system that observes an observation target OB (in vivo) using an endoscope. As illustrated in FIG. 1, the medical observation system 1 includes an insertion unit 2, a light source device 3, a light guide 4, a camera head 5, a first transmission cable 6, a display device 7, a second transmission cable 8, a control device 9, and a third transmission cable 10.

The insertion unit 2 corresponds to an optical viewing tube according to the present disclosure. In the first embodiment, the insertion unit 2 is implemented by a rigid endoscope. That is, the insertion unit 2 has an elongated shape that is entirely rigid, or partially rigid with a partially flexible portion, so as to be inserted into an observation target OB The insertion unit 2 includes an optical system having one or more lenses and configured to collect second light (subject image) described below from the observation target OB.

In addition, at a proximal end (eyepiece 21) of the insertion unit 2, there is provided an excitation light cut filter 22 (FIG. 1) that partially, substantially, or completely suppresses excitation light (described below) included in the collected second light (subject image).

The excitation light cut filter 22 may be disposed in the camera head 5, not limited to the insertion unit 2.

The light source device 3 is connected with one end of the light guide 4. The light source device 3 supplies light to the one end of the light guide 4 under the control of the control device 9. As illustrated in FIG. 1, the light source device 3 includes a first light source 31 and a second light source 32.

The first light source 31 emits first light (broadband light such as white light, or narrowband light such as red light, green light, or blue light) including at least a part of a visible light wavelength band. Examples of the configuration of the first light source 31 include a configuration including a white light emitting diode (LED), and a configuration including three light sources individually emit red light, green light, and blue light, and an optical element that combines the red light, the green light, and the blue light. The first light source 31 may include an LED or a semiconductor laser. The number of the first light sources 31 may be one or in plurality.

The second light source 32 emits excitation light that excites a substance contained in the observation target OB. The second light source 32 may be constructed with an LED or a semiconductor laser. The number of the second light sources 32 may be one or in plurality. Furthermore, the second light source 32 may emit, for example, infrared light or ultraviolet light.

Here, examples of the substance contained in the observation target OB to be excited by the excitation light include an agent or a fluorescent dye applied to the observation target OB or a fluorescent substance derived from the observation target OB, being a substance constituting the observation target OB.

Examples of the above-described agent given to the observation target OB include "5-ALA (PP-IX)", "ADS780WS", "ADS830WS", "aggregation-induced emission dots allophycocyanin (APC)", "boron-dipyrromethane (BODIPY)", "CLR 1502", "Flavins", "fluorescamine", "fluorescein", "fluoro-gold", "green fluorescence protein", "indocyanine green (ICG)", "IRDye 78", "IR-PEG nanoparticles", "Isothiocyanate", "rose Bengal", "SGM-101", and "trypan blue".

In addition, examples of the above-described fluorescent dyes to be applied to the observation target OB include "coumarine", "Cy3", "DyLight547", "GE3126", "metal nanoclusters", "oxacarbocyanine", "Rhodamine", "Riboflavin", "fluorescein", "AlexaFluor660", "AlexaFluor680", "AlexaFluor700", "Cy5", "Cy5.5", "Dy677", "Dy682", "Dy752", "Dy-Light647", "HiLyte Fluor 647", "HiLyte Fluor 680", "IRDye 700DX", "methylene blue", "Porphyrins", "Porphysomes", "VivoTag-680", "VivoTag-S680", "AlexaFluor750", "AlexaFluor790", "carbocyanine", "conjugated copolymers", "CW800-CA", "Cy7", "Cy7.5", "cyanine dyes", "Dy780", "HiLyte Fluor 750", "Indocarbocyanine", "IR-786", "IRDye 800CW", "IRDye 800RS", "IRDye 800BK", "Nervelight", "OTL-38 (Pafolacianine)", "Polymethine", "VivoTag-S750", "Xanthene", and "LUM-015".

Furthermore, examples of the fluorescent substance derived from the observation target OB constituting the observation target OB itself include "collagen", "elastin", and "NADH".

In the first embodiment, the light source device 3 is separated from the control device 9. However, the configuration is not limited to this, and it is allowable to adopt a configuration in which the light source device 3 is provided inside the same housing as that for the control device 9.

The light guide 4 has its one end detachably connected to the light source device 3. The light guide 4 has its other end detachably connected to the insertion unit 2. The light guide 4 transmits the light (first light and excitation light) supplied from the light source device 3 from one end to the other end and supplies the light to the insertion unit 2. The light (first light or excitation light) supplied to the insertion unit 2 is emitted from a distal end of the insertion unit 2 and applied to the observation target OB. When the first light is applied to the observation target OB, return light of the first light (reflected light of the first light) from the observation target OB is condensed by the optical system in the insertion unit 2. In addition, when the excitation light is applied to the observation target OB, the return light of the excitation light from the observation target OB is condensed by the optical system in the insertion unit 2. The return light of the excitation light includes: the excitation light reflected by the observation target OB; and fluorescence (hereinafter, denoted as observation target fluorescence) emitted from a substance contained in the observation target OB when the excitation light is applied to the observation target OB to excite the substance.

Hereinafter, for convenience of description, the above-described return light of the first light from the observation target OB and return light of the excitation light from the observation target OB will be denoted as second light.

The camera head 5 corresponds to an imaging device according to the present disclosure. The camera head 5 is detachably connected to the eyepiece 21 of the insertion unit 2. Under the control of the control device 9, the camera head 5 captures the second light condensed by the insertion unit 2 and generates a pixel signal. Hereinafter, for convenience of description, the pixel signal may be denoted as a captured image.

The detailed configuration of the camera head 5 will be described in "Configuration of camera head" described below.

The first transmission cable 6 has its one end detachably connected to the control device 9 via a connector CN1 (FIG. 1). The first transmission cable 6 has its other end detachably connected to the camera head 5 via a connector CN2 (FIG. 1). The first transmission cable 6 transmits the captured image or the like output from the camera head 5 to the control device 9, and transmits a control signal, a synchronization signal, a clock, power, or the like output from the control device 9 to the camera head 5 individually.

The captured image and the like transmitted from the camera head 5 to the control device 9 via the first transmission cable 6 may be transmitted as an optical signal or may be transmitted as an electric signal. The similar applies to transmission of the control signal, the synchronization signal, and the clock from the control device 9 to the camera head 5 via the first transmission cable 6.

The display device 7 is implemented by a display using liquid crystal, organic Electro Luminescence (EL), or the like, and displays an image based on a video signal from the control device 9 under the control of the control device 9.

The second transmission cable 8 has its one end detachably connected to the display device 7. The second transmission cable 8 has its other end detachably connected to the control device 9. The second transmission cable 8 transmits the video signal processed by the control device 9 to the display device 7.

The control device 9 corresponds to the medical control device according to the present disclosure. The control device 9 is implemented by a central processing unit (CPU), a Field-Programmable Gate Array (FPGA), or the like, and comprehensively controls operation of the light source device 3, the camera head 5, and the display device 7.

A detailed configuration of the control device 9 will be described in "Configuration of control device" described below.

The third transmission cable 10 has its one end detachably connected to the light source device 3. The third transmission cable 10 has its other end detachably connected to the control device 9. The third transmission cable 10 transmits the control signal from the control device 9 to the light source device 3.

[Configuration of camera head]

Next, a configuration of the camera head 5 will be described.

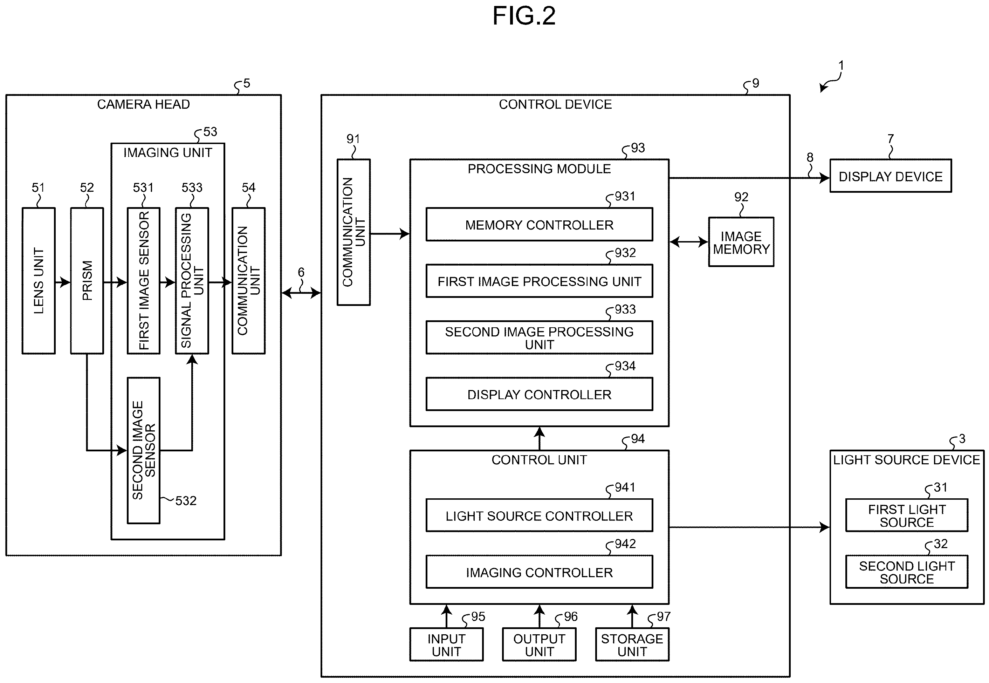

FIG. 2 is a block diagram illustrating a configuration of the camera head 5 and the control device 9.

As illustrated in FIG. 2, the camera head 5 includes a lens unit 51, a prism 52, an imaging unit 53, and a communication unit 54.

The lens unit 51 includes one or more lenses. The lens unit 51 forms an image of the second light (subject image) condensed by the insertion unit 2 on each of imaging surfaces of the first and second image sensors 531 and 532.

The prism 52 separates the second light (subject image) through the lens unit 51 into light of the first and second wavelength bands different from each other. The light of the first wavelength band is light of a wavelength band excluding at least a part of the wavelength band of the observation target fluorescence, and is light including at least a part of the wavelength band of visible light. Hereinafter, the light in the first wavelength band will be denoted as a first subject image. The light in the second wavelength band is light in a wavelength band excluding at least a part of the wavelength band of visible light, and is light including at least a part of the wavelength band of the observation target fluorescence. Hereinafter, the light in the second wavelength band will be denoted as a second subject image. The prism 52 allows the first subject image to travel toward the first image sensor 531. The prism 52 allows the second subject image to travel toward the second image sensor 532.

The imaging unit 53 images the observation target OB under the control of the control device 9. As illustrated in FIG. 2, the imaging unit 53 includes the first image sensor 531, the second image sensor 532, and a signal processing unit 533.

The first and second image sensors 531 and 532 each receive a subject image and convert the subject image into an electric signal (analog signal). In the first embodiment, each of the first and second image sensors 531 and 532 is formed with a Complementary Metal Oxide Semiconductor (CMOS) which is a rolling shutter type image sensor including a plurality of pixels two-dimensionally arranged in units of horizontal lines.

Here, although not specifically illustrated, the first image sensor 531 includes: an ineffective area in which an output signal is not used to generate a captured image; an optical black area (OB area); and an effective pixel area that converts the first subject image formed by the lens unit 51 into a pixel signal and outputs the pixel signal. The second image sensor 532 similarly includes an ineffective area, an optical black area (OB area), and an effective pixel area.

Under the control of the control device 9, the first image sensor 531 captures the first subject image via the prism 52. That is, the first image sensor 531 images light including at least a part of the wavelength band of visible light. Hereinafter, for convenience of description, a captured image generated by capturing the first subject image by the first image sensor 531 will be denoted as a normal light image.

Under the control of the control device 9, the second image sensor 532 captures the second subject image via the prism 52. That is, the second image sensor 532 captures an image of light including at least a part of the wavelength band of the observation target fluorescence. Hereinafter, for convenience of description, a captured image generated by capturing the second subject image by the second image sensor 532 will be denoted as a fluorescence image.

The number of pixels of the normal light image and the number of pixels of the fluorescence image may be different from each other or may be the same.

Under the control of the control device 9, the signal processing unit 533 performs signal processing on the captured image (analog signal) generated by the first and second image sensors 531 and 532 and outputs the captured image (digital signal)).

For example, the signal processing unit 533 performs signal processing such as processing of removing reset noise, processing of multiplying an analog gain to amplify the analog signal, A/D conversion, or the like on the captured image (analog signal) generated by the first and second image sensors 531 and 532.

The communication unit 54 functions as a transmitter that sequentially transmits the captured image output from the imaging unit 53 to the control device 9 via the first transmission cable 6. For example, the communication unit 54 includes a high-speed serial interface that performs captured image communication with the control device 9 via the first transmission cable 6 at a transmission rate of 1 Gbps or more.

The communication unit 54 may alternately transmit or may simultaneously transmit the normal light image and the fluorescence image to the control device 9.

[Configuration of control device]

Next, the configuration of the control device 9 will be described with reference to FIG. 2.

As illustrated in FIG. 2, the control device 9 includes a communication unit 91, image memory 92, a processing module 93, a control unit 94, an input unit 95, an output unit 96, and a storage unit 97.

The communication unit 91 functions as a receiver that receives captured images sequentially transmitted from the camera head 5 (communication unit 54) via the first transmission cable 6. For example, the communication unit 91 includes a high-speed serial interface that performs captured image communication with the communication unit 54 at a transmission rate of 1 Gbps or more.

The image memory 92 includes Dynamic Random Access Memory (DRAM) or the like. The image memory 92 can temporarily store a plurality of frames of captured images sequentially output from the camera head 5 (communication unit 54).

Under the control of the control unit 94, the processing module 93 processes the captured image sequentially transmitted from the camera head 5 (communication unit 54) and received by the communication unit 91. As illustrated in FIG. 2, the processing module 93 includes a memory controller 931, a first image processing unit 932, a second image processing unit 933, and a display controller 934.

The memory controller 931 controls writing of a captured image into the image memory 92 and reading of the captured image from the image memory 92. More specifically, the memory controller 931 controls to write the normal light image received by the communication unit 91 in the image memory 92, and controls to read the normal light image from the image memory 92 at a specific timing to be input to the first image processing unit 932. In addition, the memory controller 931 controls to write the fluorescence image received by the communication unit 91 in the image memory 92, controls to read the fluorescence image from the image memory 92 at a specific timing so as to be input to the second image processing unit 933.

The first image processing unit 932 executes first image processing on the input normal light image.

Examples of the first image processing include optical black subtraction processing (clamping), white balance adjustment processing, demosaic processing, color correction matrix processing, gamma correction processing, YC processing of converting RGB signals into luminance chrominance signals (Y and Cb/Cr signals), digital gain adjustment of multiplying the digital gain, noise removal, and filter processing of structure enhancement.

The second image processing unit 933 executes second image processing on the input fluorescence image.

Examples of the second image processing include optical black subtraction processing (clamping), white balance adjustment processing, demosaic processing, color correction matrix processing, gamma correction processing, YC processing of converting RGB signals into luminance chrominance signals (Y and Cb/Cr signals), digital gain adjustment of multiplying the digital gain, noise removal, and filter processing of structure enhancement.

The first and second image processing may be mutually different image processing, or may be the same image processing.

Under the control of the control unit 94, the display controller 934 generates a video signal for displaying the normal light image that has undergone the first image processing executed by the first image processing unit 932 and the fluorescence image that has undergone the second image processing executed by the second image processing unit 933. Subsequently, the display controller 934 outputs the video signal to the display device 7 via the second transmission cable 8.

The control unit 94 is implemented by executing various programs stored in the storage unit 97 by a controller such as a CPU or a micro processing unit (MPU). The control unit 94 controls the operations of the light source device 3, the camera head 5, and the display device 7 and controls the entire operation of the control device 9. The control unit 94 may include an integrated circuit such as an application specific integrated circuit (ASIC) or an FPGA, not limited to the CPU or the MPU. As illustrated in FIG. 2, the control unit 94 has functions as a light source controller 941 and an imaging controller 942.

Note that the functions of the light source controller 941 and the imaging controller 942 will be described in "Functions of light source controller and imaging controller" described below.

The input unit 95 corresponds to an operation receiving unit according to the present disclosure. The input unit 95 includes an operation device such as a mouse, a keyboard, and a touch panel, and receives user operations performed by a user such as a practitioner. Subsequently, the input unit 95 outputs an operation signal corresponding to the user operation to the control unit 94.

The output unit 96 includes a speaker, a printer, or the like, and outputs various types of information.

The storage unit 97 stores a program executed by the control unit 94, information needed for processing performed by the control unit 94, or the like.

[Functions of light source controller and imaging controller]

Next, functions of the light source controller 941 and the imaging controller 942 will be described.

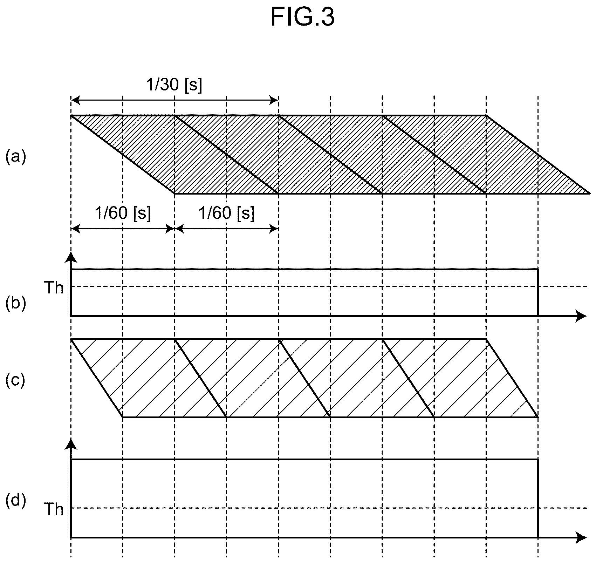

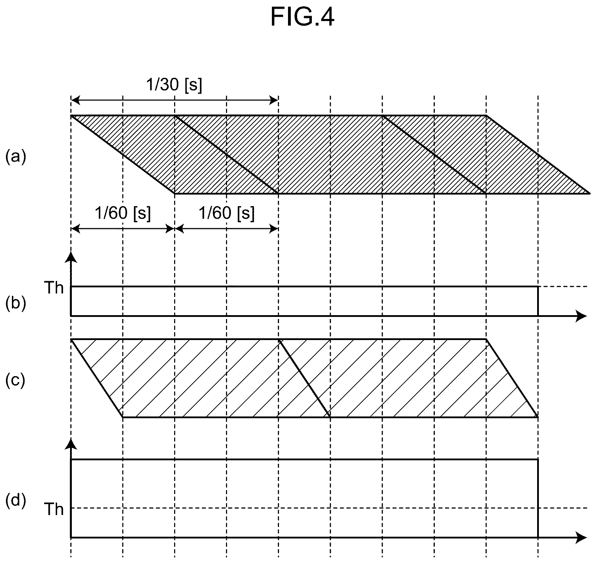

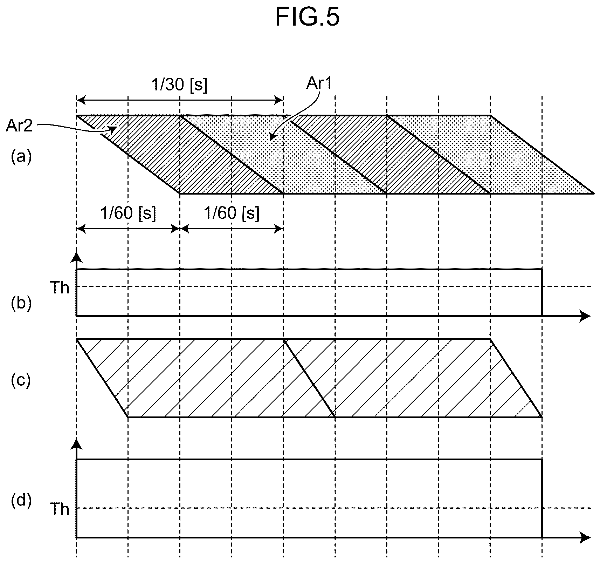

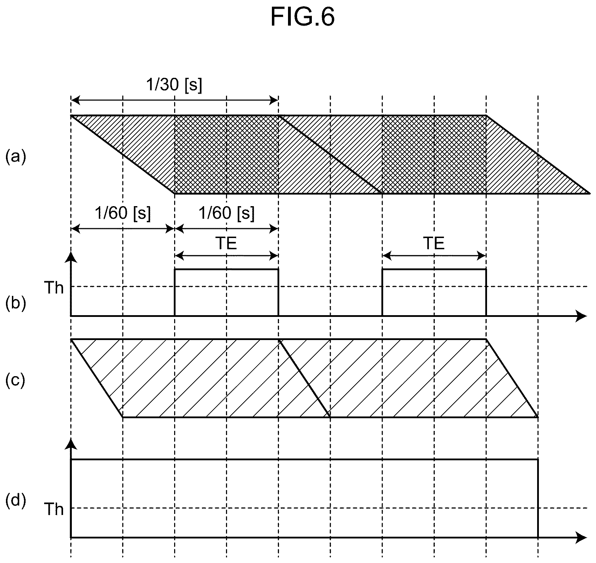

FIGS. 3 to 6 are diagrams illustrating the functions of the light source controller 941 and the imaging controller 942. Specifically, FIG. 3 is a diagram illustrating first light source control and first imaging control. FIGS. 4 and 5 are diagrams illustrating second light source control and second imaging control. FIG. 6 is a diagram illustrating third light source control and third imaging control. Here, (a) of FIG. 3, (a) of FIG. 4, (a) of FIG. 5, and (a) of FIG. 6 are diagrams illustrating imaging control of the first image sensor 531, where the vertical axis represents the horizontal line of the first image sensor 531 (the uppermost line indicates the uppermost horizontal line (the first horizontal line), and the lowermost line indicates the lowermost horizontal line (the last line)), and the horizontal axis represents time. The parallelogram area is an area that contributes to generation of a normal light image in one field. (b) of FIG. 3, (b) of FIG. 4, (b) of FIG. 5, and (b) of FIG. 6 are diagrams illustrating lighting control, where a vertical axis represents a power value[W] to be supplied to the first light source 31, and a horizontal axis represents time (supply time of power to be supplied to the first light source 31). In the first embodiment, because the voltage value supplied to the first light source 31 is fixed, the vertical axis corresponds to the current value supplied to the first light source 31 in (b) of FIG. 3, (b) of FIG. 4, (b) of FIG. 5, and (b) of FIG. 6. (c) of FIG. 3, (c) of FIG. 4, (c) of FIG. 5, and (c) of FIG. 6 are diagrams illustrating imaging control of the second image sensor 532, where the vertical axis represents the horizontal line of the second image sensor 532 (the uppermost line indicates the uppermost horizontal line (the first horizontal line), and the lowermost line indicates the lowermost horizontal line (the last line)), and the horizontal axis represents time. The parallelogram area is an area that contributes to generation of a fluorescence image in one field. (d) of FIG. 3, (d) of FIG. 4, (d) of FIG. 5, and (d) of FIG. 6 are diagrams illustrating lighting control, where the vertical axis represents the power value[W] to be supplied to the second light source 32, and the horizontal axis represents time (supply time of power to be supplied to the second light source 32). In the first embodiment, since the voltage value supplied to second light source 32 is fixed, the vertical axis corresponds to the current value supplied to second light source 32 in (d) of FIG. 3, (d) of FIG. 4, (d) of FIG. 5, and (d) of FIG. 6.

The light source controller 941 can execute each of the first to third light source controls. Furthermore, the imaging controller 942 can execute each of the first to third imaging controls.

Hereinafter, the "first light source control and first imaging control", the "second light source control and second imaging control", and the "third light source control and third imaging control" will be described in order.

[First light source control and first imaging control]

First, the first light source control and the first imaging control will be described with reference to FIG. 3.

The imaging controller 942 performs first imaging control using a method typically referred to as a rolling shutter method of sequentially starting exposure of the first and second image sensors 531 and 532 in one field period for each horizontal line, and sequentially performing reading for each horizontal line for which a predetermined period (also referred to as a shutter speed) has elapsed from the start of exposure. Furthermore, in the first imaging control of the NTSC system, one field is set to 1/60[s] ((a) of FIG. 3 and (c) of FIG. 3).

The light source controller 941 executes first light source control of controlling the operation of the first and second light sources 31 and 32. Here, the first light source control includes a first control of constantly turning on (continuously turning on) the first light source 31 and a second control of constantly turning on the second light source 32 ((b) of FIG. 3 and (d) of FIG. 3). Furthermore, in the first light source control, the current value to be supplied to the first light source 31 is adjusted based on the brightness (average value of luminance values, etc.) of a specific area (detection area) in the normal light image in order to adjust the normal light image to the reference brightness. Furthermore, the first light source control also executes lighting control of maintaining the light amount ratio as in the following.

That is, the light source controller 941 adjusts the brightness of the normal light image as described above, and adjusts the brightness of the fluorescence image with reference to ratio information indicating the light amount ratio stored in the storage unit 97. Specifically, the light source controller 941 refers to the ratio information and adjusts the current value to be supplied to the second light source 32 in accordance with the adjustment of the current value to be supplied to the first light source 31 so as to maintain a state in which the ratio between the light amount of the first light emitted from the first light source 31 and the light amount of the excitation light to be emitted from the second light source 32 is a specific ratio.

[Second light source control and second imaging control]

Next, the second light source control and the second imaging control will be described with reference to FIGS. 4 and 5.

Meanwhile, since the observation target fluorescence from the observation target OB is weak, the signal value based on the observation target fluorescence is very low, in the fluorescence image. Accordingly, in order to increase the signal value, it is conceivable to perform long-time exposure in which a plurality of fields are artificially set as one field ((a) of FIG. 4 and (c) of FIG. 4). The examples in (a) of FIG. 4 and (c) of FIG. 4 are examples in which two fields are artificially set as one field, and the one field is set to 1/30[s].

However, in a case where the above-described long-time exposure and the above-described lighting control for maintaining the light amount ratio are used in combination, the following problems may occur.

That is, execution of the long-time exposure will increase not only the signal level in the fluorescence image but also the signal level in the normal light image (an increase of about twice in the example of FIG. 4). That is, since the brightness of the normal light image becomes high, when the lighting control for maintaining the light amount ratio is executed, the light amount of the first light is decreased in order to adjust the brightness of the normal light image to the reference brightness, and the light amount of the excitation light is also decreased in accordance with the decrease in the light amount of the first light. This leads to a first problem of a decrease in the brightness of the fluorescence image.

In order to solve the first problem, the following method is considered.

As illustrated in (b) of FIG. 4, power of N/2[W], which is half the power of N[W] assumed to be supplied to the first light source 31, is supplied to the first light source 31. With this method, the normal light image does not become brighter than necessary, and the brightness of the fluorescence image does not decrease even when the lighting control for maintaining the light amount ratio is executed. However, this method has a second problem described below.

Since the light amount of the first light emitted from the first light source 31 is greatly reduced, the margin of the current value (the margin from a drive lower limit value Th) for adjusting the light amount of the first light emitted from the first light source 31 is to be reduced. Furthermore, in the lighting control of maintaining the light amount ratio, the light amount of the excitation light is adjusted in accordance with the light amount of the first light, and this decreases also the margin of the current value (the margin from the drive lower limit value Th) for adjusting the light amount of the excitation light to be emitted from the second light source 32.

The second light source control and the second imaging control are methods of solving the second problem.

The second imaging control is control of discarding image information generated by the first image sensor 531 in accordance with light reception in a partial period of the exposure period of the first image sensor 531. In the first embodiment, the second imaging control is control of adjusting the diaphragmatic amount of the electronic shutter of the first image sensor 531. That is, in the first embodiment, the image information corresponds to the charge accumulated in each pixel by the first image sensor 531. In the example of FIG. 5, the imaging controller 942 fixes the diaphragmatic amount of the electronic shutter of the first image sensor 531 to 1/60[s]. In (a) of FIG. 5, an area Ar1 being a dot area is an area indicating sweep-out of charges by the electronic shutter. Furthermore, an area Ar2 being a hatched area is an area indicating an effective exposure period.

The second light source control is the same control as the first light source control described above.

[Third light source control and third imaging control]

Hereinafter, the optical path of the first light and the excitation light following the route through the light source device 3, the light guide 4, the insertion unit 2, to the observation target OB, is defined as a first optical path P1 (FIG. 1). In addition, an optical path of the second light following the route through the observation target OB, the insertion unit 2, to the first image sensor 531, is defined as a second optical path P2 (FIG. 1). The first and second optical paths P1 and P2 are collectively defined as an observation optical path P0 (FIG. 1).

When the first light and the excitation light propagate through the first optical path P1 and when the second light propagates through the second optical path P2, the member forming the observation optical path P0 (hereinafter, denoted as autofluorescence generating member) is irradiated with the first light, the excitation light, and the second light, whereby autofluorescence (hereinafter, denoted as unnecessary light) is generated from the autofluorescence generating member. Examples of the autofluorescence generating member include a component contained in multicomponent glass such as a lens, an organic material component contained in a color filter, an adhesive that bonds a lens and a lens, and oil attached to an optical element such as a lens. Such unnecessary light has a wavelength band including a wavelength band of observation target fluorescence, being observation target light in fluorescence observation, and will be noise in performing the fluorescence observation. Such unnecessary light is also generated by irradiating the observation target OB with the first light or the excitation light.

In a case where the observation target fluorescence from the observation target OB is weak, it is necessary to adjust a signal value based on the observation target fluorescence imaged by the second image sensor 532 in order to separate the observation target fluorescence from the unnecessary light to perform satisfactory fluorescence observation. However, it is difficult to adjust the signal value based on the observation target fluorescence because of the effects of the following (1) to (4).

(1) Agents

Typically, the light amount of observation target fluorescence emitted from an agent varies depending on the type and dosage of the agent.

The type of agent to be administered to the observation target OB is selected in accordance with the observation target OB (such as cancer, blood, and lymph). Furthermore, in order to image the observation target fluorescence emitted from the agent in the medical observation system 1, the agent to be selected is an agent capable of separating the wavelength of the excitation light for exciting the agent and the wavelength of the observation target fluorescence emitted from the agent. The agents selected in this manner have different light amounts of the observation target fluorescence emitted.

In addition, although the light amount of the observation target fluorescence can be adjusted by the dosage of the agent, it is difficult to increase the dosage more than necessary in order to achieve a minimally invasive treatment.

That is, it is difficult to adjust the light amount of the observation target fluorescence by selecting the type of agent and adjusting the dosage. As a result, it is difficult to adjust the signal value based on the observation target fluorescence imaged by the second image sensor 532 by selecting the type of agent and adjusting the dosage.

(2) Observation target

The light amount of the observation target fluorescence emitted from the observation target OB varies depending on the site/state of the observation target OB.

Specifically, in the case of the observation target OB being the site and having the state in which the agent easily stagnates, the light amount of the observation target fluorescence emitted from the observation target OB increases. In contrast, in the case of the observation target OB being the site and having the state in which the agent easily flows and hardly stagnates, the light amount of the observation target fluorescence emitted from the observation target OB decreases, together with a decrease in the afterglow time. Furthermore, in a case where the observation target OB is a tumor, the light amount of the observation target fluorescence received by the second image sensor 532 varies depending on the spread, size, and depth of the tumor.

That is, it is difficult to adjust the light amount of the observation target fluorescence depending on the type and state of the observation target OB. As a result, it is difficult to adjust the signal value based on the observation target fluorescence imaged by the second image sensor 532 depending on the type and state of the observation target OB.

(3) Light source device

The light amount of the observation target fluorescence varies depending on the light amount of the excitation light emitted from the light source device 3.

In order to increase the light amount of the excitation light, it may be necessary to adjust the power supplied to the light source device 3. However, the power that can be supplied to the light source device 3 is limited with the upper limit value of the power for operating the entire medical observation system 1. In addition, the light amount of the excitation light is required to be adjusted in consideration of conditions such as: heat generation (for example, heat generation between the location such as the light guide 4 and the insertion unit 2) in a member constituting an optical path of the excitation light; conformity to the laser class; an amount of light energy received by the observation target OB or a living body in the vicinity (there is a risk of causing a burn when the amount of light energy is large); or a fading speed of observation target fluorescence emitted from an agent. The light amount of the excitation light can also be adjusted by changing the number of light sources of the excitation light mounted on the light source device 3. However, the number of light sources may affect the size of the light source device 3. The size of the light source device 3 may be limited by the size of a cart for carrying the light source device 3 or the like.

That is, it is difficult to adjust the light amount of the observation target fluorescence by adjusting the light amount of the excitation light. As a result, it is difficult to adjust the signal value based on the observation target fluorescence imaged by the second image sensor 532 by adjusting the light amount of the excitation light.

(4) Image sensor

The signal value based on the observation target fluorescence generated from the second image sensor 532 differs depending on the light amount of the observation target fluorescence received by the second image sensor 532.

In order to adjust the signal value based on the observation target fluorescence, it is desired to select the second image sensor 532 having optimum sensitivity, configuration, and the like for imaging the observation target fluorescence. However, the second image sensor 532 might be required not only to output an image for fluorescence observation based on reception of observation target fluorescence in a predetermined wavelength band, but also to output an image for normal light observation based on reception of visible light such as white light. Furthermore, the second image sensor 532 might also be required to output an image for fluorescence observation corresponding to a wide wavelength band or a plurality of wavelength bands among wavelength bands including visible light and invisible light. Furthermore, even if the same agent is used, the light amount of the observation target fluorescence might change depending on the procedure or the observation target OB. Therefore, the second image sensor 532 might also be required to output an image for fluorescence observation corresponding to such a change in the light amount of the observation target fluorescence.

In that case, the second image sensor 532 needs to select a sensor element capable of supporting these observations, leading to a case of not being able to use an image sensor having optimum characteristics for imaging fluorescence in a predetermined wavelength band. Furthermore, the second image sensor 532 is disposed in the camera head 5, and since the camera head 5 has a size and weight suitable for observation, the type including the size of the second image sensor 532 might be limited. Not only in the configuration in which the second image sensor 532 is disposed in the camera head 5, but also in a case where the second image sensor is disposed at the distal end of a rigid endoscope or a flexible endoscope, the second image sensor has a size and weight suitable for observation, leading to the limitation of the type including the size of the second image sensor 532.

That is, it is difficult to adjust the signal value based on the observation target fluorescence imaged by the second image sensor 532 by selecting the type of the second image sensor 532.

As described above, the signal value based on the observation target fluorescence imaged by the second image sensor 532 is determined within the above constraint, and thus cannot be easily adjusted.

In the "first light source control and first imaging control" and the "second light source control and second imaging control" described above, the first and second light sources 31 and 32 are constantly turned on, leading to high likelihood of generation of unnecessary light. The third light source control and the third imaging control are control of partially, substantially, or completely suppressing unnecessary light.

As illustrated in (a) of FIG. 6 and (c) of FIG. 6, the third imaging control is control based on the second imaging control but in which the adjustment of the diaphragmatic amount of the electronic shutter is not performed. That is, the third imaging control is control to perform the long-time exposure alone.

The third light source control includes: second control ((d) of FIG. 6) of constantly turning on the second light source 32; and a first control of turning on the first light source 31 only within a full line exposure period TE (1/60[s] in the example of FIG. 6) in the first image sensor 531. In the example of FIG. 6, the lighting period of the first light source 31 is set to 1/60[s] in the first control, but may be set to other periods, for example, 1/120[s]. That is, by reducing the period during which the first light is emitted in the first control, generation of unnecessary light is partially, substantially, or completely suppressed. Here, the reduction of the emission period of the first light decreases the brightness of the normal light image, but the decreased brightness is increased by the first image processing (digital gain adjustment or the like) performed by the first image processing unit 932. That is, the first and second light source controls have an advantage that the brightness of the normal light image can be increased without performing digital gain adjustment. Similarly to the first and second light source control, the third light source control also performs the lighting control of maintaining the light amount ratio.

[Method of setting lighting period of the first light source 31 in third light source control]

Next, a method of setting a lighting period of the first light source 31 in the third light source control will be described.

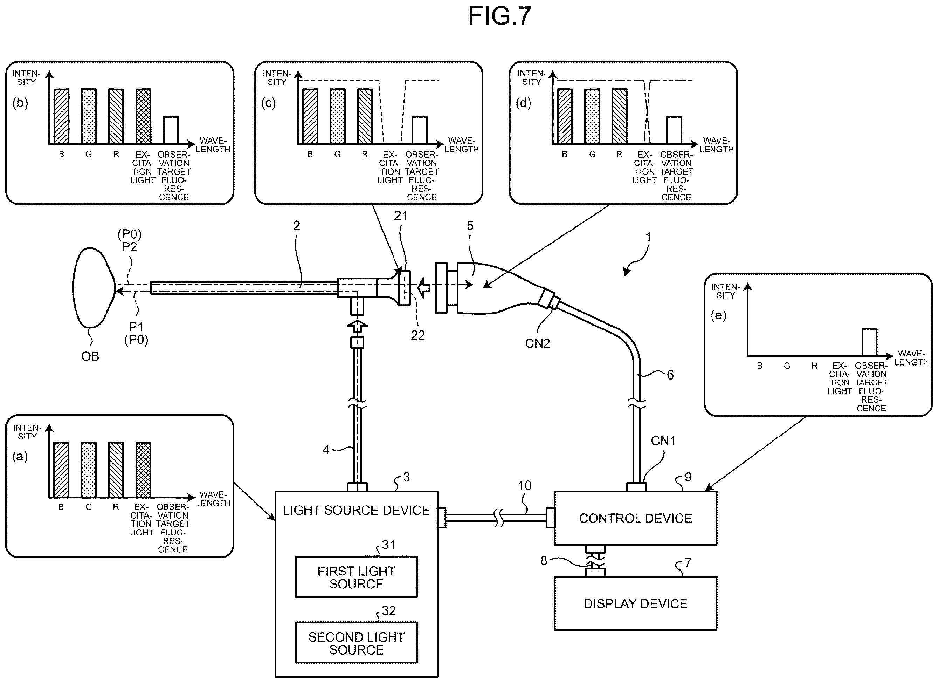

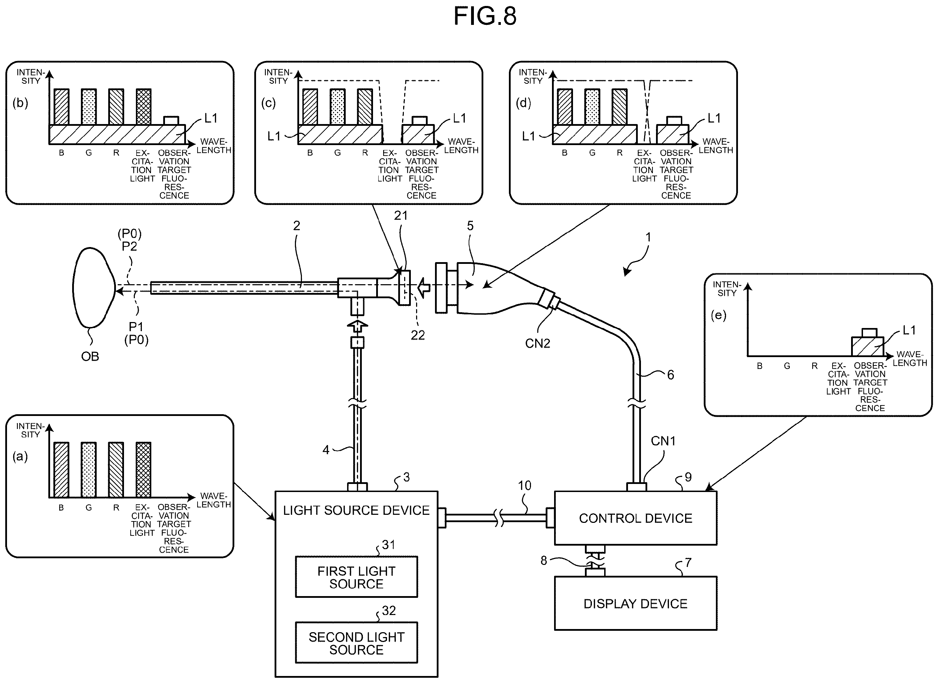

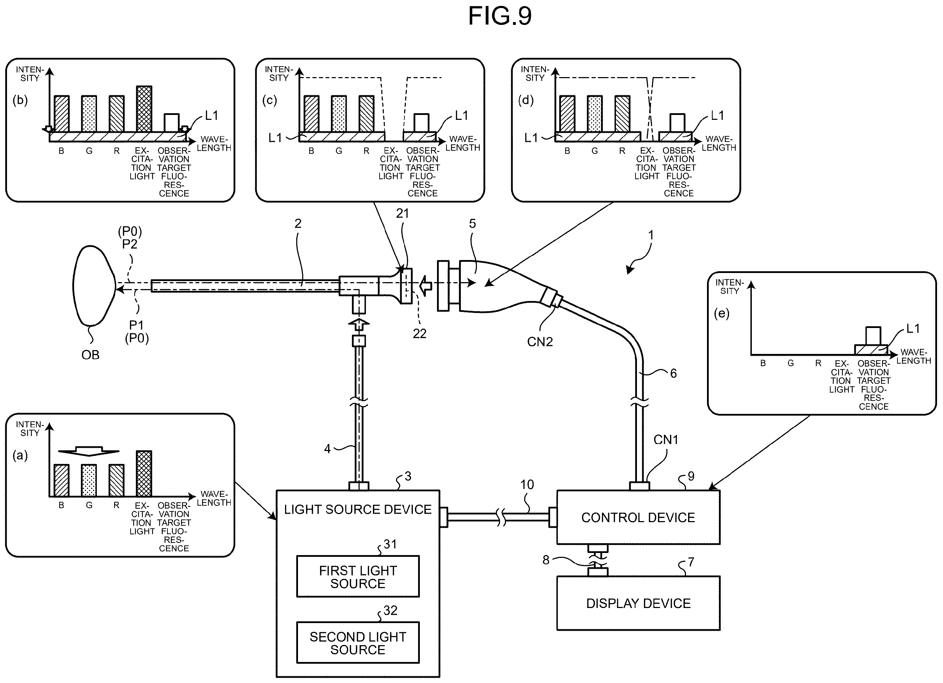

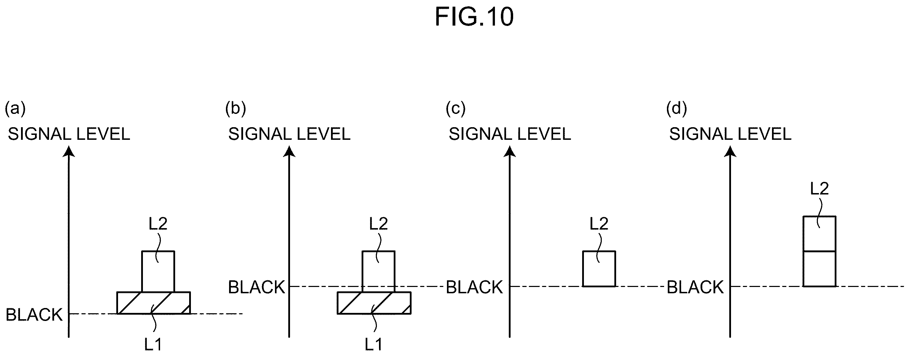

FIGS. 7 to 10 are diagrams illustrating a method of setting a lighting period of the first light source 31 in the third light source control. Specifically, FIG. 7 is a diagram corresponding to FIG. 1, and is a diagram illustrating an ideal state with no generation of unnecessary light to be noise. FIG. 8 is a diagram corresponding to FIG. 7, and is a diagram illustrating an actual state in which unnecessary light to be noise is generated. FIG. 9 is a diagram illustrating a case where the third light source control and the third imaging control are executed in the state of FIG. 8. FIG. 10 is a diagram illustrating second image processing (clamping and digital gain adjustment) by the second image processing unit 933.

In FIGS. 7 to 9, the first light source 31 is configured to emit each light of red light, green light, and blue light. In addition, red light is denoted as "R", green light is denoted as "G", and blue light is denoted as "B".

First, an ideal state of no generation of unnecessary light to be noise will be described with reference to FIG. 7.

As illustrated in (a) of FIG. 7, light source device 3 emits each light of first light (R, G, and B) and excitation light. In FIG. 7, for convenience of description, the first light (R, G, and B) emitted from the light source device 3 and the excitation light have same intensity.

When the observation target OB is irradiated with the first light (R, G, and B) and the excitation light via the first optical path P1, the substance contained in the observation target OB is excited by the excitation light, and the observation target fluorescence is emitted from the substance as illustrated in (b) of FIG. 7. In FIG. 7, for convenience of description, the intensity of the observation target fluorescence is illustrated as about half of the intensity of the first light (R, G, and B) and the intensity of the excitation light. However, the actual intensity is about 1/20 with an agent that causes emission of bright observation target fluorescence and is about 1/500 with an agent that causes emission of dark observation target fluorescence.

When the second light (first light (R, G, and B), excitation light, and observation target fluorescence), which is return light of the first light (R, G, and B) and the excitation light from the observation target OB, propagates through the second optical path P2, the excitation light included in the second light is partially, substantially, or completely suppressed by the excitation light cut filter 22 as illustrated in (c) of FIG. 7.

In addition, as illustrated in (d) of FIG. 7, the second light (first light (R, G, and B) and observation target fluorescence) after passing through the excitation light cut filter 22 is separated, by the prism 52, into the first subject image including the first light (R, G, and B) and the second subject image including the observation target fluorescence ((e) of FIG. 7). The first image sensor 531 captures the first subject image and generates a normal light image. On the other hand, the second image sensor 532 captures the second subject image and generates a fluorescence image.

Subsequently, the second image processing is executed on the fluorescence image by the second image processing unit 933, so that the signal value based on the observation target fluorescence is increased by the digital gain adjustment. Furthermore, by executing the second image processing on the fluorescence image, for example, a pseudo color such as green is attached at a position where the intensity of the observation target fluorescence is relatively high. Furthermore, the display controller 934 may superimpose the normal light image after undergoing the first image processing executed by the first image processing unit 932 and the fluorescence image after undergoing the second image processing executed by the second image processing unit 933 to generate a superimposed image and may generate a video signal for displaying the superimposed image.

Next, an actual state in which unnecessary light to be noise is generated will be described with reference to FIG. 8.

When the first light (R, G, and B), the excitation light, and the second light propagate through the observation optical path P0, unnecessary light is generated from the autofluorescence generating member. In FIG. 8, reference sign "L1" indicates unnecessary light. As illustrated in (b) of FIG. 8, the unnecessary light is light in a wide wavelength range including the wavelength band of the observation target fluorescence. Therefore, as illustrated in (e) of FIG. 8, the second subject image includes the unnecessary light L1 in addition to the observation target fluorescence. On the fluorescence image, separation of the observation target fluorescence and the unnecessary light L1 is difficult.

Next, a case where the third light source control and the third imaging control are executed will be described with reference to FIG. 9.

When the third light source control is executed, as illustrated in (a) of FIG. 9, the intensity of the first light (R, G, and B) is reduced as compared with (a) of FIG. 8. This reduces, as illustrated in parts (b) to (e) of FIG. 9, the intensity of unnecessary light L1 as compared with (b) to (e) of FIG. 8. In the first embodiment, the light source controller 941 executes, in the third light source control, the first control (control to turn on the first light source 31) such that the signal value based on the observation target fluorescence becomes larger than the signal value based on the unnecessary light L1 imaged by the second image sensor 532. In other words, the light source controller 941 executes the first control such that the S/N ratio of the signal value based on the observation target fluorescence imaged by the second image sensor 532 becomes larger than 1.

A method of calculating the S/N ratio will be described in "Method of Calculating S/N Ratio" described below.

For example, in a case where the S/N ratio of the signal value based on the observation target fluorescence imaged by the second image sensor 532 is larger than 1, as illustrated in FIG. 10, the signal value based on the observation target fluorescence can be further amplified by the second image processing performed by the second image processing unit 933, making it possible to separate the signal value based on the observation target fluorescence and the signal value based on the unnecessary light L1 from each other. In FIG. 10, unnecessary light is denoted as the unnecessary light L1, and observation target fluorescence is denoted as observation target fluorescence L2.

Specifically, the second image processing unit 933 performs, in the clamping, as illustrated in (a) to (c) of FIG. 10, raising of the level (clamp value) to be cut as a black level so as to separate the signal value based on the unnecessary light L1 and the signal value based on the observation target fluorescence L2 from each other.

However, as illustrated in (c) of FIG. 10, the signal level in the signal after the increase of the clamp value is lower than that in the original state.

To handle this, as illustrated in (d) of FIG. 10, the second image processing unit 933 performs digital gain adjustment to increase the signal level in the signal illustrated in (c) of FIG. 10.

Subsequently, the light source controller 941 sets a control value in the first control or a control value in the second control based on a predetermined value according to the present disclosure. In the first embodiment, the predetermined value according to the present disclosure is the control value itself in the first control. In the first embodiment, the lighting period (the emission period of the first light) of the first light source 31 is adopted as the control value in the first control according to the present disclosure, but the control value is not limited thereto. The control value in the first control according to the present disclosure is not limited to the lighting period of the first light source 31 as long as the control value is emission energy of the first light emitted from the first light source 31, and may be a value such as a current value supplied to the first light source 31. Furthermore, a control threshold according to the present disclosure is the maximum value of the control value in the first control in which the S/N ratio of the signal value based on the observation target fluorescence L2 imaged by the second image sensor 532 is larger than 1. In the first embodiment, the light source controller 941 sets the control value in the first control of the third light source control to a control value being the control threshold or less. Furthermore, in the first embodiment, the control threshold is a period being the full line exposure period TE or less.

In the first embodiment, the predetermined value according to the present disclosure is a lighting period of the first light source 31, and is a fixed value determined in advance at a design stage such that an S/N ratio of a signal value based on the observation target fluorescence L2 imaged by the second image sensor 532 is larger than 1.

As described above, the light source controller 941 executes the first control so as to suppress the signal value based on the unnecessary light L1 imaged by the second image sensor 532, and executes the second control for a period that overlaps with at least a part of the period during which the first control is executed and is longer than the period during which the first control is executed. The control of the light source controller 941 is not limited to the above, and conversely, the first control may be executed in a period that overlaps with at least a part of the period in which the second control is executed and is shorter than the period in which the second control is executed, and the second control may be executed such that the signal value based on the unnecessary light L1 imaged by the second image sensor 532 becomes smaller than the signal value based on the observation target fluorescence L2. That is, the light source controller 941 executes at least one of the first control and the second control such that the signal value based on the observation target fluorescence L2 becomes larger than the signal value based on the unnecessary light L1 imaged by the second image sensor 532. In addition, the light source controller 941 executes at least one of the first control and the second control so as to increase a difference between the signal value based on the observation target fluorescence L2 and the signal value based on the unnecessary light L1 imaged by the second image sensor 532.

[Method of calculating S/N Ratio]

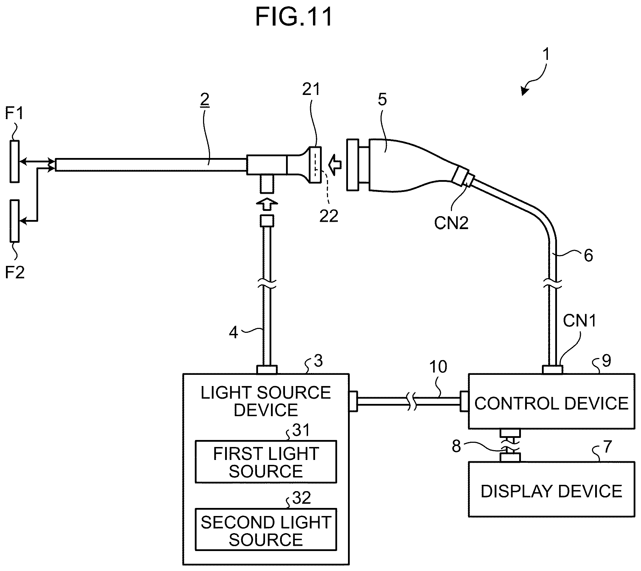

FIG. 11 is a diagram illustrating a method of calculating the S/N ratio. Specifically, FIG. 11 is a diagram corresponding to FIG. 1.

The method of calculating the S/N ratio according to the present embodiment is a method based on "2.D.8.Excitation light crosstalk" and "3.G.Excitation light crosstalk" in the title "Performance test methods for near-infrared fluorescence imaging" of Document A below.

(Document A)

"MEDICAL PHYSICS" Volume 47, Issue 8, August 2020, Pages 3389-3401

The method of calculating the S/N ratio uses first and second subjects (phantoms) F1 and F2 as illustrated in FIG. 11.

The first subject F1 is a subject (phantom) containing no agent and containing only scattering particles.

The second subject F2 is a subject (phantom) containing an agent at an appropriate concentration for a living body to be observed.

The S/N ratio is calculated by the following Formula (1).

$$\text{S/N ratio} = ((c)-(a))/((b)-(a)) \tag{1}$$

Here, (a) of Formula (1) is a luminance level based on the pixel signal output from the second image sensor 532 in a state where no external light enters the camera head 5 and the first and second light sources 31 and 32 are turned off in the medical observation system 1 illustrated in FIG. 11. Note that, (a) may be set as a luminance level based on a pixel signal output from the second image sensor 532 in a state where a mechanical shutter is disposed between the camera head 5 and the eyepiece 21, and entrance of light into the camera head 5 is prohibited by the mechanical shutter. (b) is a luminance level based on the pixel signal output from the second image sensor 532 when the first and second light sources 31 and 32 are turned on, the first subject F1 is irradiated with the first light and the excitation light, and the second light is imaged by the second image sensor 532 in the medical observation system 1 illustrated in FIG. 11. (c) is a luminance level based on the pixel signal output from the second image sensor 532 when the first and second light sources 31 and 32 are turned on, the second subject F2 is irradiated with the first light and the excitation light, and the second light is imaged by the second image sensor 532 in the medical observation system 1 illustrated in FIG. 11.

The predetermined value according to the present disclosure is preferably a value at which the S/N ratio calculated by Formula (1) using a pixel signal (RAW signal (pre-processing pixel signal according to the present invention)) before image processing (first image processing or second image processing) is executed on the pixel signal output from the second image sensor 532 is larger than 1. The predetermined value according to the present disclosure is preferably a value at which the S/N ratio calculated by Formula (1) using a pixel signal (post-processing pixel signal according to the present invention) after image processing (first image processing or second image processing) is executed on the pixel signal output from the second image sensor 532 is 4 or more.

According to the first embodiment described above, the following effects are obtained.

The control device 9 according to the first embodiment executes at least one of the first control and the second control such that the signal value based on the observation target fluorescence L2 becomes larger than the signal value based on the unnecessary light L1 imaged by the second image sensor 532 and generated at least due to the first light. That is, the control device 9 executes at least one of the first control and the second control such that the S/N ratio of the signal value based on the observation target fluorescence L2 imaged by the second image sensor 532 becomes larger than 1. This makes it possible for the control device 9 to generate an image in which the user can easily differentiate the observation target fluorescence L2 and the unnecessary light L1.

Therefore, the control device 9 according to the first embodiment achieves satisfactory fluorescence observation.

In particular, the control device 9 sets the control value in the first control as the lighting period of the first light source 31, and adjusts the control value to partially, substantially, or completely suppress the unnecessary light L1, thereby enabling the S/N ratio of the signal value based on the observation target fluorescence L2 imaged by the second image sensor 532 to be larger than 1.

Therefore, fluorescence observation can be performed satisfactorily by simple processing.

(Modification 1-1 of first embodiment)

In the first embodiment described above, a fixed value determined in advance at the design stage is adopted as the predetermined value according to the present disclosure, but the predetermined value is not limited thereto.

For example, the light source controller 941 controls to emit only the first light from the first light source 31 in the light source device 3 at the timing of executing the white balance adjustment processing before starting the observation of the observation target OB. The light source controller 941 may set a predetermined value according to the present disclosure in which the S/N ratio of the signal value based on the observation target fluorescence L2 imaged by the second image sensor 532 is larger than 1 based on the signal value of the pixel signal output from the first image sensor 531 or the second image sensor 532. For example, since generation of more unnecessary light L1 is speculated by the larger signal value of the pixel signal, the light source controller 941 decreases the predetermined value according to the present disclosure.

Even when adopting the configuration of modification 1-1 described above, it is possible to obtain the effects similar to those of the above-described first embodiment.

(Modification 1-2 of first embodiment)

In the first embodiment described above, a fixed value determined in advance at the design stage is adopted as the predetermined value according to the present disclosure, but the predetermined value is not limited thereto.

For example, the light source controller 941 may set the predetermined value according to the present disclosure such that the S/N ratio of the signal value based on the observation target fluorescence L2 imaged by the second image sensor 532 becomes larger than 1 based on the brightness (average value of luminance values, etc.) of a specific area (detection area) in the normal light image. In other words, the light source controller 941 sets the predetermined value according to the present disclosure based on the signal value of the pixel signal output from the first image sensor 531. For example, since generation of more unnecessary light L1 is speculated by the higher brightness of a specific area (detection area) in the normal light image, the light source controller 941 decreases the predetermined value according to the present disclosure.

Even when adopting the configuration of modification 1-2 described above, it is possible to obtain the effects similar to those of the above-described first embodiment.

(Modification 1-3 of first embodiment)

In the first embodiment described above, a fixed value determined in advance at the design stage is adopted as the predetermined value according to the present disclosure, but the predetermined value is not limited thereto.

For example, the light source controller 941 may set the predetermined value according to the present disclosure such that the S/N ratio of the signal value based on the observation target fluorescence L2 imaged by the second image sensor 532 becomes larger than 1 based on the brightness (average value of luminance values, etc.) of a specific area (detection area) in the fluorescence image. In other words, the light source controller 941 sets the predetermined value according to the present disclosure based on the signal value of the pixel signal output from the second image sensor 532. For example, since generation of more unnecessary light L1 is speculated by the higher brightness of a specific area (detection area) in the fluorescence image, the light source controller 941 decreases the predetermined value according to the present disclosure.

Modification 1-3 described above achieves the following effect in addition to effects similar to those of the first embodiment described above.

According to modification 1-3, the light source controller 941 can estimate the type of the agent being used based on the brightness (average value of luminance values, etc.) of a specific area (detection area) in the fluorescence image. That is, the predetermined value according to the present disclosure can be increased when using an agent that causes emission of a bright observation target fluorescence L2, and the predetermined value according to the present disclosure can be decreased when using an agent that causes emission of a dark observation target fluorescence L2, thereby achieving sufficient reduction of the unnecessary light L1 In addition, even with the same agent, the degree of accumulation of the agent varies depending on the subject to be viewed (depending on the clinical department), and thus, the signal value based on the observation target fluorescence L2 varies depending on the region to be observed. For example, since the agent (ICG) in the case of observing blood flow in the large intestine runs along the blood flow, the signal value based on the observation target fluorescence L2 becomes a signal value indicating darkness. On the other hand, since the agent is accumulated when the bile duct is observed, the signal value based on the observation target fluorescence L2 is a signal value indicating brightness. In this manner, the predetermined value according to the present disclosure can be changed based on the signal value based on the observation target fluorescence L2 (brightness (average value of luminance values, etc.) of a specific area (detection area) in the fluorescence image).

(Modification 1-4 of first embodiment)

In the first embodiment described above, a fixed value determined in advance at the design stage is adopted as the predetermined value according to the present disclosure, but the predetermined value is not limited thereto.

For example, the above-described fixed value (predetermined value according to the present disclosure) may be changed in accordance with a user operation on the input unit 95.

Even when adopting the configuration of modification 1-4 described above, it is possible to obtain the effects similar to those of the above-described first embodiment.

(Modification 1-5 of first embodiment)

In the first embodiment described above, a fixed value determined in advance at the design stage is adopted as the predetermined value according to the present disclosure, but the predetermined value is not limited thereto.

For example, the light source controller 941 may set the predetermined value according to the present disclosure based on the fading time of the signal value based on the observation target fluorescence L2 imaged by the second image sensor 532. In other words, the light source controller 941 determines the type of the agent being used, and sets the predetermined value according to the present disclosure depending on the type of the agent. For example, the light source controller 941 sets the predetermined value according to the present disclosure such that the value is to be smaller in a case where the type of the agent is determined to be the agent that causes emission of the dark observation target fluorescence L2 than in a case where the type of the agent is determined to be the agent that causes emission of the bright observation target fluorescence L2.

Even when adopting the configuration of modification 1-5 described above, it is possible to obtain the effects similar to those of the above-described first embodiment.

(Second embodiment)

Next, a second embodiment will be described.

In the following, identical reference numerals are given to the components similar to those in the first embodiment described above, and detailed description thereof will be omitted or simplified.

In the following, identical reference numerals are given to the components similar to those in the first embodiment described above, and detailed description thereof will be omitted or simplified.

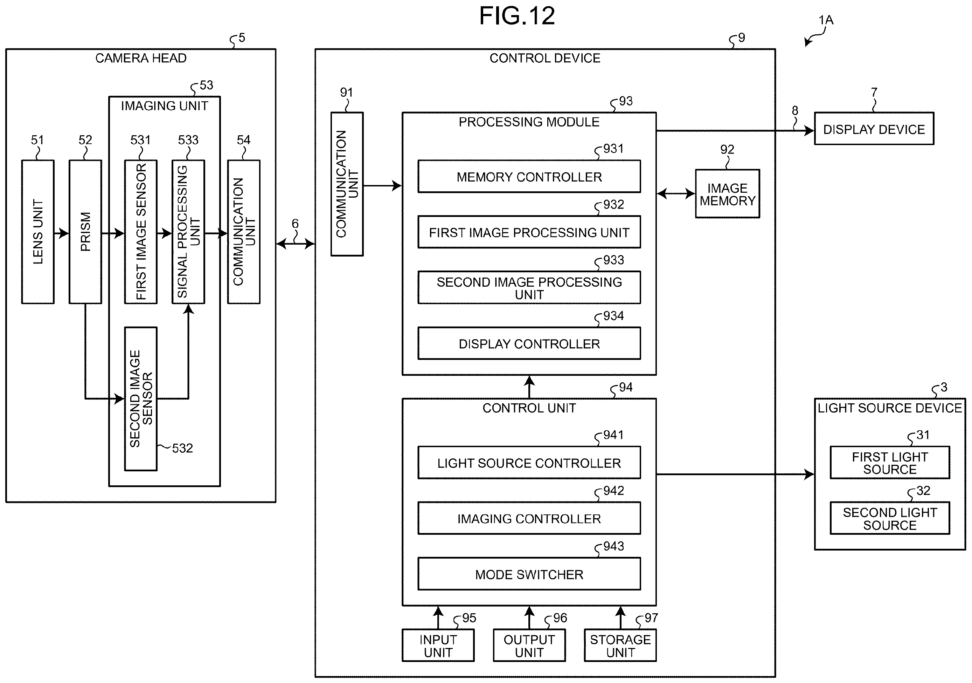

FIG. 12 is a diagram illustrating a configuration of a medical observation system 1A according to the second embodiment. Specifically, FIG. 12 is a diagram corresponding to FIG. 2.

As illustrated in FIG. 12, the medical observation system 1A according to the second embodiment is different from the medical observation system 1 described in the first embodiment in that a mode switcher 943 is added to the control unit 94.

The mode switcher 943 switches the medical observation system 1A to a first mode or a second mode in accordance with a user operation on the input unit 95.

The first mode is a mode of partially, substantially, or completely suppressing unnecessary light L1. In the first mode, the light source controller 941 executes the third light source control. Furthermore, the imaging controller 942 executes the third imaging control.

The second mode is a mode of not suppressing unnecessary light L1 partially, substantially, or completely. In the second mode, the light source controller 941 executes the first light source control or the second light source control. The imaging controller 942 executes the first imaging control or the second imaging control. That is, in the second mode, the light source controller 941 sets the control value in the first control of the third light source control to a control value larger than the control threshold. Incidentally, the second mode may be a mode of partially, substantially, or completely suppressing the unnecessary light L1, similarly to the first mode. That is, the light source controller 941 may execute the third light source control in the second mode. Furthermore, the imaging controller 942 may execute the third imaging control in the second mode. At this time, the second mode is a mode in which the suppression amount of the unnecessary light L1 is smaller than that in the first mode. That is, in the second mode, the control value in the first control of the third light source control is larger than the control value in the first mode.

With the above-described second embodiment, effects similar to the effects of the above-described first embodiment can be achieved.

Meanwhile, the control of partially, substantially, or completely suppressing the unnecessary light L1 has an effect of preventing the observation target fluorescence L2 from being hidden under the unnecessary light L1, but this control decreases the brightness of the normal light image and increases noisiness or the like even with adjustment of the brightness of the normal light image by digital gain adjustment. In addition, some type of agent enables sufficiently bright emission of the observation target fluorescence L2, and enables the observation to be performed without partially, substantially, or completely suppressing the unnecessary light L1.

The control device 9 according to the second embodiment switches between the first mode and the second mode described above. Therefore, the first mode can be selected when the unnecessary light L1 needs to be partially, substantially, or completely suppressed, while the second mode can be selected when the unnecessary light L1 does not need to be partially, substantially, or completely suppressed, leading to improvement of the convenience.

(Modification 2-1 of second embodiment)

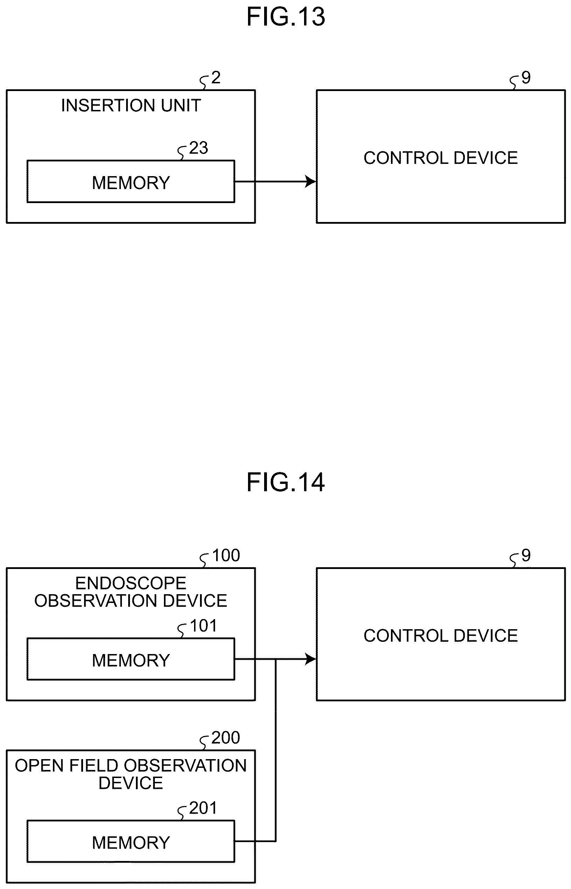

FIG. 13 is a diagram illustrating modification 2-1 of the second embodiment.

In the second embodiment described above, the mode switcher 943 switches the medical observation system 1A to the first mode or the second mode in accordance with the user operation on the input unit 95, but the mode switching is not limited thereto.

As illustrated in FIG. 13, the mode switcher 943 according to modification 2-1 reads an endoscope Identifier (ID) stored in memory 23 provided in the insertion unit 2, and switches the medical observation system 1A to the first mode or the second mode based on the endoscope ID. The endoscope ID, being identification information unique to the insertion unit 2, is to be different information depending on the diameter of the insertion unit 2, the type of the excitation light cut filter 22, the glass material of the lens constituting the optical system in the insertion unit 2, and the like.

Modification 2-1 described above achieves the following effect in addition to effects similar to those of the second embodiment described above.

The generation amount of the unnecessary light L1 varies depending on the diameter of the insertion unit 2, the type of the excitation light cut filter 22, the glass material of the lens constituting the optical system in the insertion unit 2, and the like.

The control device 9 according to modification 2-1 switches the medical observation system 1A to the first mode or the second mode based on the endoscope ID. This makes it possible to appropriately switch to the first mode or the second mode in accordance with the type of the insertion unit 2.

Note that, in the above-described first embodiment, the predetermined value according to the present disclosure may be set based on the endoscope ID, as in modification 2-1. For example, the light source controller 941 sets a predetermined value according to the present disclosure such that the value is to be smaller when connected with the insertion unit 2 (endoscope ID) having a larger amount of unnecessary light L1 than when being connected with the insertion unit 2 (endoscope ID) having a smaller amount of unnecessary light L1.

(Modification 2-2 of second embodiment)

FIG. 14 is a diagram illustrating modification 2-2 of the second embodiment.

In the second embodiment described above, the mode switcher 943 switches the medical observation system 1A to the first mode or the second mode in accordance with the user operation on the input unit 95, but the mode switching is not limited thereto.

As illustrated in FIG. 14, the control device 9 according to modification 2-2 can be connected to an endoscope observation device 100 and an open field observation device 200 individually.

The endoscope observation device 100 is a device using an endoscope, and is a device including the insertion unit 2 and the camera head 5 described in the second embodiment.

On the other hand, the open field observation device 200 is a device used in laparotomy.

Subsequently, the mode switcher 943 according to modification 2-2 reads the device ID stored in memory 101 provided in the endoscope observation device 100 connected to the control device 9 or reads the device ID stored in memory 201 provided in the open field observation device 200 connected to the control device 9. The device ID is identification information indicating whether it is an endoscope observation device or an open field observation device. When having determined that the connected device is the open field observation device 200 based on the read device ID, the mode switcher 943 switches the medical observation system 1A to the second mode. When having determined that the connected device is the endoscope observation device 100 based on the read device ID, the mode switcher 943 switches the medical observation system 1A to the first mode.

Modification 2-2 described above achieves the following effect in addition to effects similar to those of the second embodiment described above.

Meanwhile, in the use of the open field observation device 200, turning into the first mode in which the first light blinks allows the user to directly view the blinking of the first light, which is not preferable.

The control device 9 according to modification 2-2 switches the medical observation system 1A to the first mode or the second mode based on device ID. This makes it possible to avoid a situation of turning into the first mode when the open field observation device 200 is used, leading to improvement of convenience.

Note that, in the above-described first embodiment, the predetermined value according to the present disclosure may be set based on the device ID, as in modification 2-2. For example, the light source controller 941 sets a predetermined value according to the present disclosure such that the value is to be larger when the open field observation device 200 is connected than when the endoscope observation device 100 is connected.

(Modification 2-3 of second embodiment)

In the second embodiment described above, the mode switcher 943 switches the medical observation system 1A to the first mode or the second mode in accordance with the user operation on the input unit 95, but the mode switching is not limited thereto.

For example, the light source controller 941 controls to emit only the first light from the first light source 31 in the light source device 3 at the timing of executing the white balance adjustment processing before starting the observation of the observation target OB. The mode switcher 943 may switch the medical observation system 1A to the first mode or the second mode based on the signal value of the pixel signal output from the first image sensor 531 or the second image sensor 532. For example, in a case where the signal value of the pixel signal is larger than a predetermined threshold, it is speculated that a large amount of unnecessary light L1 is generated, and thus, the mode switcher 943 switches the medical observation system 1A to the first mode. On the other hand, the mode switcher 943 switches the medical observation system 1A to the second mode in a case where the signal value of the pixel signal is the predetermined threshold or less.

Even when adopting the configuration of modification 2-3 described above, it is possible to obtain the effects similar to those of the above-described second embodiment.

(Modification 2-4 of second embodiment)

In the second embodiment described above, the mode switcher 943 switches the medical observation system 1A to the first mode or the second mode in accordance with the user operation on the input unit 95, but the mode switching is not limited thereto.

For example, the mode switcher 943 may switch the medical observation system 1A to the first mode or the second mode based on the brightness (average value of luminance values or the like) of a specific area (detection area) in the normal light image. In other words, the light source controller 941 may switch the medical observation system 1A to the first mode or the second mode based on the signal value of the pixel signal output from the first image sensor 531. For example, in a case where the brightness of a specific area (detection area) in the normal light image is higher than a predetermined threshold (brightness), it is speculated that a large amount of unnecessary light L1 is generated, and thus, the mode switcher 943 switches the medical observation system 1A to the first mode. On the other hand, in a case where the brightness of a specific area (detection area) in the normal light image is the predetermined threshold or less, the mode switcher 943 switches the medical observation system 1A to the second mode.

Even when adopting the configuration of modification 2-4 described above, it is possible to obtain the effects similar to those of the above-described second embodiment.

(Modification 2-5 of second embodiment)

In the second embodiment described above, the mode switcher 943 switches the medical observation system 1A to the first mode or the second mode in accordance with the user operation on the input unit 95, but the mode switching is not limited thereto.

For example, the mode switcher 943 may switch the medical observation system 1A to the first mode or the second mode based on the brightness (average value of luminance values or the like) of a specific area (detection area) in the fluorescence image. In other words, the mode switcher 943 may switch the medical observation system 1A to the first mode or the second mode based on the signal value of the pixel signal output from the second image sensor 532. For example, in a case where the brightness of a specific area (detection area) in the fluorescence image is higher than a predetermined threshold (brightness), it is speculated that a large amount of unnecessary light L1 is generated, and thus, the mode switcher 943 switches the medical observation system 1A to the first mode. On the other hand, in a case where the brightness of a specific area (detection area) in the fluorescence image is the predetermined threshold or less, the mode switcher 943 switches the medical observation system 1A to the second mode.

Even when adopting the configuration of modification 2-5 described above, it is possible to obtain the effects similar to those of the above-described second embodiment.

(Modification 2-6 of second embodiment)

In the second embodiment described above, the mode switcher 943 switches the medical observation system 1A to the first mode or the second mode in accordance with the user operation on the input unit 95, but the mode switching is not limited thereto.

For example, the mode switcher 943 may switch the medical observation system 1A to the first mode or the second mode based on the fading time of the signal value based on the observation target fluorescence L2 imaged by the second image sensor 532. In other words, the mode switcher 943 determines the type of the agent being used, and switches the medical observation system 1A to the first mode or the second mode depending on the type of the agent. For example, when having determined that the agent is an agent that causes emission of the bright observation target fluorescence L2, the light source controller 941 switches the medical observation system 1A to the second mode. On the other hand, when having determined that the agent is an agent that causes emission of the dark observation target fluorescence L2, the light source controller 941 switches the medical observation system 1A to the first mode.

Even when adopting the configuration of modification 2-6 described above, it is possible to obtain the effects similar to those of the above-described second embodiment.

(Modification 2-7 of second embodiment)

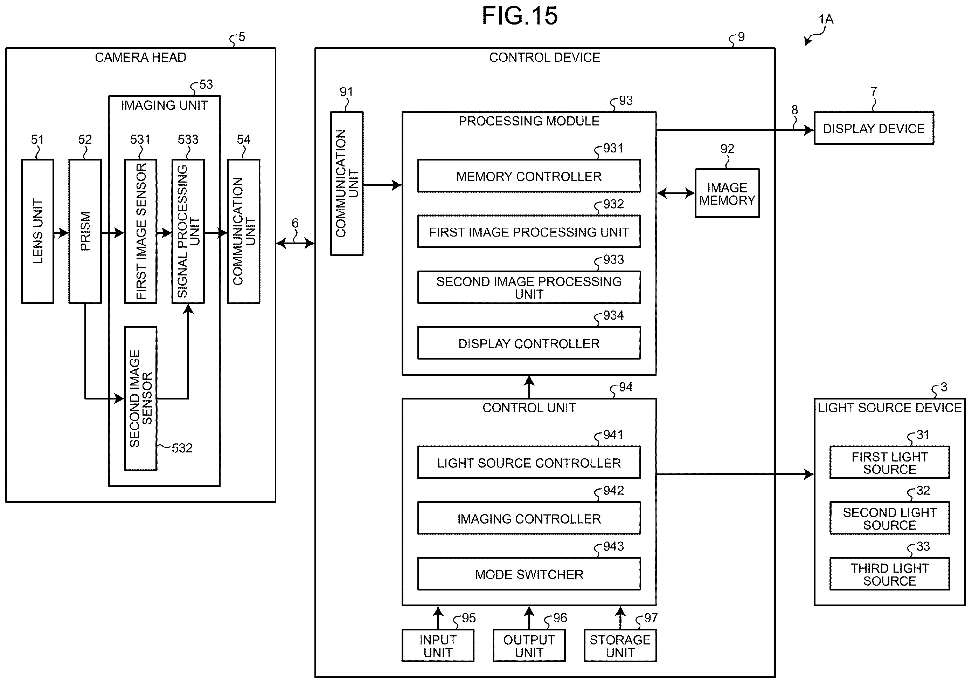

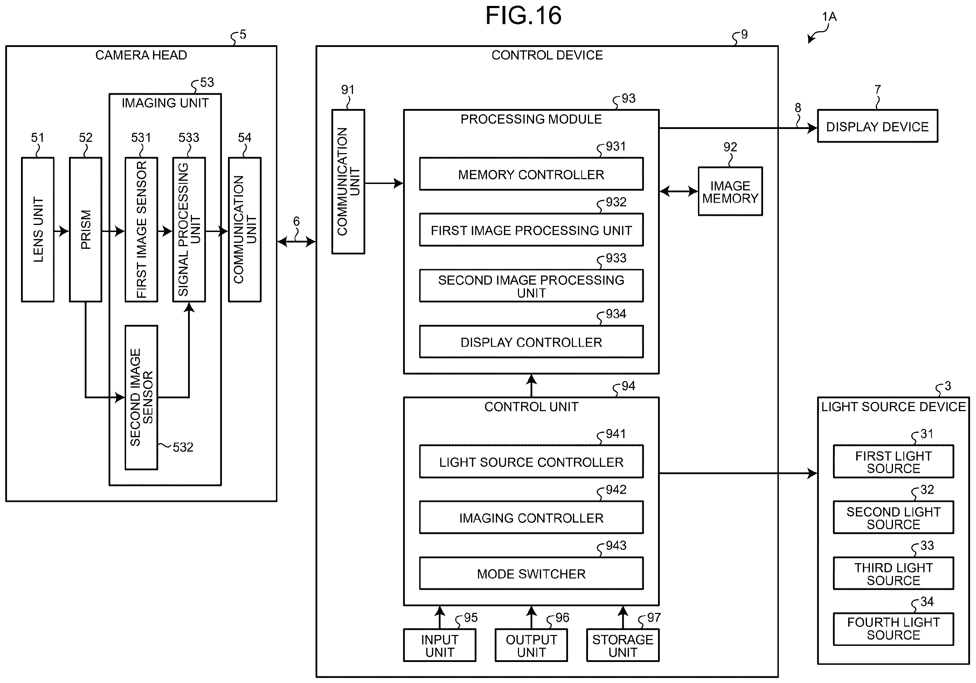

FIGS. 15 and 16 are diagrams illustrating modification 2-7 of the second embodiment. Specifically, FIGS. 15 and 16 are diagrams corresponding to FIG. 1.

The configuration described in the second embodiment above may be applied to surgery or the like using a plurality of agents illustrated in Table 1 below. In Table 1, "○" is attached to an agent to be used, and "×" is attached to an agent not to be used.

Table 1

| Category | Target disease | Agent | | | | |
|---|---|---|---|---|---|---|
| | | Fluorescein | ALM-488 | LUM-015 | 5-ALA | ICG |
| Surgery | Lung cancer | × | ○ | × | × | ○ |
| | Colorectal cancer | | | × | | |
| | Pediatric brain tumor | | | × | | |
| | Breast cancer | | | ○ | | |
| Photo dynamic diagnosis | Brain tumor treatment | | | × | ○ | × |
| | Superficial urothelial cancer | ○ | × | | | |

Here, fluorescein emits observation target fluorescence L2 of about 520 [nm] when being irradiated with excitation light in a wavelength band of about 470 [nm] to 480 [nm]. ALM-488 emits observation target fluorescence L2 in a wavelength band of about 530 [nm] when being irradiated with excitation light in a wavelength band of about 488 [nm]. LUM-015 emits observation target fluorescence L2 in a wavelength band of about 675 [nm] when being irradiated with excitation light in a wavelength band of about 650 [nm]. 5-ALA emits observation target fluorescence L2 in a wavelength band of about 530 [nm] to 630 [nm] when being irradiated with excitation light in a wavelength band of about 405 [nm]. ICG emits observation target fluorescence L2 in a wavelength band of about 830 [nm] when being irradiated with excitation light in a wavelength band of about 805 [nm].

For example, when lymph node dissection is performed in lung cancer lobectomy, ALM-488 and ICG are used as illustrated in Table 1. That is, ALM-488 is used to visualize the left recurrent laryngeal nerve while ICG is used to visualize a lymph node. By such visualization of the left recurrent laryngeal nerve and the lymph node, it is possible to differentiate the left recurrent laryngeal nerve and the lymph nodes from each other and reduce recurrent laryngeal nerve palsy when performing a lobectomy. At this time, since two agents of the ALM-488 and the ICG are used, there is a need, as illustrated in FIG. 15, to use the medical observation system 1A having a configuration including not only the second light source 32 that emits excitation light for exciting one of the two agents but also a third light source 33 that emits excitation light for exciting the other of the two agents. The second and third light sources 32 and 33 correspond to the second light source according to the present disclosure. The medical observation system 1A switches to the first mode in a case of using one agent (corresponding to a first agent according to the present disclosure) that causes weak emission of the observation target fluorescence L2 among the two agents of the ALM-488 and the ICG, and switches to the second mode in a case of using the other agent (corresponding to a second agent according to the present disclosure) among the two. The doctor who performs the lobectomy can grasp the position of one of the left recurrent laryngeal nerve and the lymph node from the image displayed on the display device 7 in the first mode, and can grasp the position of the other of the left recurrent laryngeal nerve and the lymph node from the image displayed on the display device 7 in the second mode.

For example, in proctectomy of colorectal cancer, when lateral node dissection is performed, the ALM-488 and the ICG are used as illustrated in Table 1. That is, the ALM-488 is used to visualize the neurovascular bundle and the ICG is used to visualize the lymph node. By such visualization of the neurovascular bundle and the lymph node, it is possible to differentiate the neurovascular bundle and the lymph nodes from each other, and reduce urination disorder, dyschezia, male dysfunction, motor dysfunction, or the like due to nerve damage during the proctectomy. At this time, since two agents of the ALM-488 and the ICG are used, there is a need, as illustrated in FIG. 15, to use the medical observation system 1A having a configuration including not only the second light source 32 that emits excitation light for exciting one of the two agents but also a third light source 33 that emits excitation light for exciting the other of the two agents. The second and third light sources 32 and 33 correspond to the second light source according to the present disclosure. The medical observation system 1A switches to the first mode in a case of using one agent (corresponding to a first agent according to the present disclosure) that causes weak emission of the observation target fluorescence L2 among the two agents of the ALM-488 and the ICG, and switches to the second mode in a case of using the other agent (corresponding to a second agent according to the present disclosure) among the two. The doctor who performs the proctectomy can grasp the position of one of the neurovascular bundle and the lymph node from the image displayed on the display device 7 in the first mode, and can grasp the position of the other of the neurovascular bundle and the lymph node from the image displayed on the display device 7 in the second mode.

For example, in malignant tumor resection of a pediatric brain tumor, the ALM-488 and the ICG are used when the resection of a malignant tumor is performed as illustrated in Table 1. That is, ALM-488 is used to visualize the nerves while ICG is used to visualize vessels (blood flow). By such visualization of nerves and blood vessels, it is possible to differentiate a malignant tumor from nerves and blood vessels, to prevent recurrence of cancer after performing the malignant tumor resection, and to prevent damage to nerves or blood vessels when performing the malignant tumor resection. At this time, since two agents of the ALM-488 and the ICG are used, there is a need, as illustrated in FIG. 15, to use the medical observation system 1A having a configuration including not only the second light source 32 that emits excitation light for exciting one of the two agents but also a third light source 33 that emits excitation light for exciting the other of the two agents. The second and third light sources 32 and 33 correspond to the second light source according to the present disclosure. The medical observation system 1A switches to the first mode in a case of using one agent (corresponding to a first agent according to the present disclosure) that causes weak emission of the observation target fluorescence L2 among the two agents of the ALM-488 and the ICG, and switches to the second mode in a case of using the other agent (corresponding to a second agent according to the present disclosure) among the two. The doctor performing the malignant tumor resection can grasp the position of one of the nerve and the blood vessel from the image displayed on the display device 7 in the first mode, and can grasp the position of the other of the nerve and the blood vessel from the image displayed on the display device 7 in the second mode.

When malignant tumor resection or axillary lymph node dissection is performed in mastectomy of breast cancer, for example, ALM-488, LUM-015, and ICG are used as illustrated in Table 1. That is, ALM-488 is used to visualize the nerves such as the intercostal brachial nerve and the thoracic dorsal nerve, LUM-015 is used to visualize a malignant tumor, and ICG is used to visualize a lymph node. Such visualization of the nerve, the malignant tumor, and the lymph node makes it possible to differentiate the malignant tumor, the nerve, and the lymph node from each other, leading to reduction of damage to the nerve when performing mastectomy. At this time, since three agents of the ALM-488, the LUM-015, and the ICG are used, there is a need, as illustrated in FIG. 16, to use the medical observation system 1A having a configuration including not only the second light source 32 that emits excitation light that excites one of the three agents, but also the third light source 33 that emits excitation light that excites one of the other two agents and a fourth light source 34 that emits excitation light that excites the other of the other two agents. The second to fourth light sources 32 to 34 correspond to the second light source according to the present disclosure. The medical observation system 1A switches to the first mode in a case of using an agent (corresponding to a first agent according to the present disclosure) that causes weak emission of the observation target fluorescence L2 among the three agents of the ALM-488, the LUM-015, and the ICG, and switches to the second mode in a case of using the other agents (corresponding to the second agent according to the present disclosure) among the three. The doctor who performs the mastectomy can grasp the position of at least one of the nerve, the malignant tumor, and the lymph node from the image displayed on the display device 7 in the first mode, and can grasp the other positions of the nerve, the malignant tumor, and the lymph node from the image displayed on the display device 7 in the second mode.

Furthermore, for example, when brain tumor treatment using Photo Dynamic Diagnosis (PDD) is performed, ALM-488 and 5-ALA (5-aminolevulinic acid) are used as illustrated in Table 1. That is, fluorescein being a fluorescent dye including ALM-488 is used to visualize the vicinity of the tumor, and protoporphyrin (PpIX) biosynthesized from 5-ALA is used to visualize tumor cells. By such visualization of tumor cells including the vicinity of the tumor, it is possible to prevent damage when brain tumor treatment is performed, leading to safe extraction of the tumor. At this time, since two agents of the ALM-488 and the 5-ALA are used, there is a need, as illustrated in FIG. 15, to use the medical observation system 1A having a configuration including not only the second light source 32 that emits excitation light for exciting one of the two agents but also a third light source 33 that emits excitation light for exciting the other of the two agents. The second and third light sources 32 and 33 correspond to the second light source according to the present disclosure. In addition, the medical observation system 1A switches to the first mode in a case of using 5-ALA (corresponding to the first agent according to the present disclosure) in which the observation target fluorescence L2 is weak, and switches to the second mode in a case of using the ALM-488 (corresponding to the second agent according to the present disclosure), of the two agents of the ALM-488 and the 5-ALA. The doctor who performs the brain tumor treatment can grasp the position of the tumor cell from the image displayed on the display device 7 in the first mode, and can grasp the position of the vicinity of the tumor from the image displayed on the display device 7 in the second mode.

For example, when transurethral cystectomy is performed in surgical treatment of superficial urothelial cancer using Photo Dynamic Diagnosis (PDD), fluorescein and 5-ALA are used as illustrated in Table 1. Specifically, tumors are visualized by PpIX biosynthesized from 5-ALA. Here, PpIX has a disadvantage of visualizing not only tumors but also highly metabolically inflamed tissues present in the bladder. To overcome this, false positives of highly metabolically inflamed tissues caused by PpIX are to be reduced by fluorescein. At this time, since two agents of the fluorescein and the 5-ALA are used, there is a need, as illustrated in FIG. 15, to use the medical observation system 1A having a configuration including not only the second light source 32 that emits excitation light for exciting one of the two agents but also a third light source 33 that emits excitation light for exciting the other of the two agents. The second and third light sources 32 and 33 correspond to the second light source according to the present disclosure. In addition, the medical observation system 1A switches to the first mode in a case of using 5-ALA (corresponding to the first agent according to the present disclosure) in which the observation target fluorescence L2 is weak, and switches to the second mode in a case of using the fluorescein (corresponding to the second agent according to the present disclosure), of the two agents of the fluorescein and the 5-ALA. The doctor who performs transurethral cystectomy grasps the position of the tumor from the image displayed on the display device 7 in the first mode, and grasps the false positive of the hypermetabolic inflammatory tissue due to PpIX from the image displayed on the display device 7 in the second mode.

Even when adopting the configuration of modification 2-7 described above, it is possible to obtain the effects similar to those of the above-described second embodiment.

(Modification 2-8 of second embodiment)

The configuration described in the second embodiment above may be applied to the following first to sixth scenes. Although the following first to sixth scenes adopt ICG as an agent to be used, the scenes may adopt other agents.

[First scene]

Examples of the scene in which the observation target fluorescence L2 becomes weak when ICG is used include a scene of operating on a pediatric patient.
The dosage of ICG per dose needs to be set in accordance with the patient's body weight. Accordingly, as compared with an adult patient, when ICG is administered to a pediatric patient, it is necessary to reduce the ICG in proportion to the body weight. That is, when ICG is administered to a pediatric patient, the concentration of ICG is low, and the observation target fluorescence L2 may be weak in some cases.
The medical observation system 1A switches to the first mode in a case of performing surgery on a pediatric patient (corresponding to a first surgical scene according to the present disclosure), and switches to the second mode in a case of performing surgery on an adult patient (corresponding to a second surgical scene according to the present disclosure).

[Second scene]

Examples of a scene in which the observation target fluorescence L2 becomes weak when ICG is used include a scene of performing an intrabronchial injection method.
The intrabronchial injection method is a method of intersegmental identification in segmentectomy of the lung. Specifically, in the intrabronchial injection method, ICG is sprayed to the segmental bronchus using a bronchoscope, and the lung segment to be resected is identified by fluorescence observation. That is, in the intrabronchial injection method, it is difficult to increase the concentration of ICG as compared with the case of identifying the lung segment to be resected by the ICG intravenous injection method in which ICG is intravenously injected, and the observation target fluorescence L2 may be weak in some cases.
The medical observation system 1A switches the mode to the first mode in a case where the lung segment is identified by the intrabronchial injection method (corresponding to the first surgical scene according to the present disclosure), and switches the mode to the second mode in a case where the lung segment is identified by the ICG intravenous injection method (corresponding to the second surgical scene according to the present disclosure).

[Third scene]

Examples of a scene where the observation target fluorescence L2 becomes weak when ICG is used include a scene of using a small-diameter endoscope having a reduced outer diameter of the insertion unit 2.
As fluorescence observation using ICG becomes common in the lower gastrointestinal tract, there is an increasing need to perform less invasive surgery. Under conditions with spatial constraints such as Trans-anal total mesorectal excision and single-incision surgery, a small-diameter endoscope is often used to improve operability of forceps or the like. However, the small-diameter endoscope has a narrower light guide path of the excitation light as compared with a normal endoscope or a large-diameter endoscope having a larger outer diameter of the insertion unit 2, lowering the energy of the emitted excitation light to make the observation target fluorescence L2 weak in some cases.
The medical observation system 1A switches to the first mode in a case where the small-diameter endoscope is used (corresponding to the first surgical scene according to the present disclosure), and switches to the second mode in a case where a normal endoscope or a large-diameter endoscope is used (corresponding to the second surgical scene according to the present disclosure).

[Fourth scene]

Examples of the scene in which the observation target fluorescence L2 becomes weak in the case of using ICG include a scene of using a low concentration ICG for the first time among a plurality of times when the lung segment is identified by a plurality of times of fluorescence observation by an ICG intravenous injection method.

The ICG intravenous injection method has a problem that once ICG is injected intravenously, the ICG continues to emit luminescence on the blood flow and cannot be observed again until it is metabolized in the liver. For example, if the entire lung emits luminescence due to an erroneous hemostatic site or loose ligation when segmentectomy of the lung lobe is performed, it becomes very difficult to perform segment identification again by the ICG intravenous injection method. In addition, since there is an upper limit to the adult dosage, it is difficult to perform segment identification again by the ICG intravenous injection method after ICG is metabolized.

To handle this, when the segment of the lung is first identified, the concentration of ICG is set to a low concentration (for example, a concentration of 1/10 of a normal concentration), the ICG is intravenously injected to perform fluorescence observation. When an appropriate segment cannot be identified by the fluorescence observation, the ICG is injected intravenously again with the concentration of ICG being set to a normal concentration, and fluorescence observation is performed. When identifying the lung segment for the first time, the concentration of ICG is set to a low concentration, and thus the observation target fluorescence L2 may be weak in some cases as compared with the case where the concentration of ICG is set to a normal concentration.

The medical observation system 1A switches to the first mode in a case where fluorescence observation is performed with the concentration of ICG set to a low concentration (corresponding to a first surgical scene according to the present disclosure), and switches to the second mode in a case where fluorescence observation is performed with the concentration of ICG set to a normal concentration (corresponding to a second surgical scene according to the present disclosure). By performing fluorescence observation a plurality of times at the same site in this manner, the lung segment can be identified with higher accuracy.

[Fifth scene]

Examples of the scene in which the observation target fluorescence L2 becomes weak in the case of using ICG include a scene in which a low concentration of ICG is used for the first time among a plurality of fluorescence observations when confirming the blood flow of intestinal anastomosis failure by the ICG intravenous injection method.

In a case where the blood flow of intestinal anastomosis failure is confirmed by fluorescence observation, when the blood flow cannot be appropriately confirmed due to a setting error of a device, a subject buried in tissue such as fat, or the like, it takes time until ICG is metabolized, making it difficult to confirm the blood flow again by the ICG injection method.

Therefore, similarly to the fourth scene described above, when blood flow is to be confirmed for the first time, the concentration of ICG is set to a low concentration (for example, a concentration of 1/10 of the normal concentration), the ICG is intravenously injected, and fluorescence observation is performed. When the blood flow cannot be appropriately confirmed by the fluorescence observation, the concentration of ICG is set to a normal concentration, the ICG is injected intravenously again to perform fluorescence observation. The concentration of ICG is set to a low concentration when the blood flow is confirmed for the first time, the observation target fluorescence L2 may be weak in some cases as compared with a case where the concentration of ICG is set to the normal concentration.

The medical observation system 1A switches to the first mode in a case where fluorescence observation is performed with the concentration of ICG set to a low concentration (corresponding to a first surgical scene according to the present disclosure), and switches to the second mode in a case where fluorescence observation is performed with the concentration of ICG set to a normal concentration (corresponding to a second surgical scene according to the present disclosure). By performing a plurality of fluorescence observations at the same site in this manner, it is possible to reduce complications such as peritonitis due to anastomotic failure.

[Sixth scene]

Examples of a scene in which the observation target fluorescence L2 becomes weak in the case of using ICG include a scene in which a fluorescence image is subjected to fluorescence observation using a 3D image (hereinafter, denoted as 3D fluorescence observation).

When 3D fluorescence observation is performed, a binocular relay scope may be used as the insertion unit 2. In this binocular relay scope, two optical paths are arranged in parallel in the scope. In addition, each of the two optical paths has an optical system. Subsequently, the binocular relay scope captures and emits right and left eye observation light beams having parallax with each other by the two optical systems (refer to JP 6-160731 A, for example). In such a binocular relay scope, similarly to the small-diameter endoscope described in the third scene, the light guide path of the excitation light is narrowed by the two optical paths as compared with the normal endoscope. This might reduce the energy of the emitted excitation light and weaken the observation target fluorescence L2 in some cases.

In addition, 3D fluorescence observation may use two image sensors that individually capture right and left eye observation light beams, as the second image sensor 532. When using two image sensors as the second image sensor 532, the size of the image sensor is smaller than that in a case where one image sensor is used. This might reduce the light reception energy of the observation target fluorescence L2 and weaken the signal value based on the observation target fluorescence L2 in some cases.

While the above description is an exemplary case where a rigid endoscope for 3D fluorescence observation is used, even in a case where a flexible endoscope for 3D fluorescence observation is used, the observation target fluorescence L2 may be weak, or a signal value based on the observation target fluorescence L2 may be weak.

The medical observation system 1A switches to the first mode when performing surgery using an endoscope for 3D fluorescence observation (corresponding to the first surgical scene according to the present disclosure), and switches to the second mode when performing surgery using an endoscope for fluorescence observation of a fluorescence image by a 2D image (corresponding to the second surgical scene according to the present disclosure).

In the first to sixth scenes described above, the mode switcher 943 may switch the medical observation system 1A to the first mode or the second mode in accordance with the user operation on the input unit 95, similarly to the second embodiment described above, but may switch the mode as follows.

For example, the mode switcher 943 acquires patient information regarding the patient, surgery information regarding the type of surgery, or endoscope type information regarding the type of endoscope. Subsequently, the mode switcher 943 switches the medical observation system 1A to the first mode or the second mode based on the patient information, the surgery information, or the endoscope type information.

Furthermore, for example, the mode switcher 943 estimates the patient, the type of surgery, or the type of endoscope by image recognition using a trained model generated by machine learning, for example. The trained model is, for example, a learning model after learning processing for the learning model is repeatedly executed using a plurality of sets of medical information and images each including one set of medical information and an image. The medical information is information of at least one of an age of a patient, a medical department, a surgical site, a surgical status, an agent type, an agent dosage, an imaging device (diameter of rigid endoscope, rigid endoscope or ring light), and the like. Subsequently, the mode switcher 943 switches the medical observation system 1A to the first mode or the second mode based on the estimated patient, surgery type, or endoscope type.

Furthermore, while an image sensor is disposed in the endoscope or the camera head 5 to be connected, the range of fluorescence intensity that can be imaged varies depending on the difference in the sensitivity of the image sensor and system configuration. Even in such observation, the same observation performance can be obtained by switching the medical observation system 1A to the first mode or the second mode similarly to the above.

Even when adopting the configuration of modification 2-8 described above, it is possible to obtain the effects similar to those of the above-described second embodiment.

(Modification 2-9 of second embodiment)

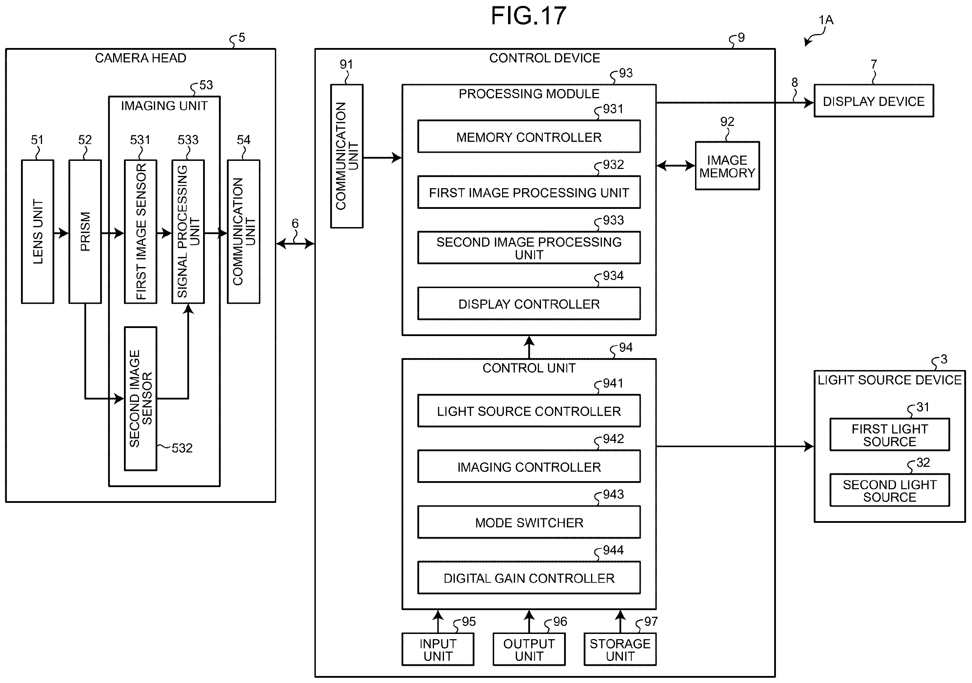

FIG. 17 is a diagram illustrating modification 2-9 of the second embodiment. Specifically, FIG. 17 is a diagram corresponding to FIG. 12.

In the second embodiment described above, the first mode may be set as a fluorescence observation mode, and the second mode may be set as a normal light observation mode.

The fluorescence observation mode (first mode) is a mode of generating a display image based on pixel signals individually output from the first and second image sensors 531 and 532. The display image is an image (hereinafter, denoted as a superimposed image) in which the normal light image and the fluorescence image are superimposed with the normal light image disposed as a background image.

The normal light observation mode (second mode) is a mode of generating a display image based on a pixel signal output from only the first image sensor 531 among the first and second image sensors 531 and 532. The display image is a normal light image.

A user such as a doctor switches between the normal light observation mode (second mode) and the fluorescence observation mode (first mode), and observes the normal light image in the normal light observation mode and the superimposed image in the fluorescence observation mode. At this time, when a phenomenon occurs in which the brightness of the normal light image in the normal light observation mode is different from the brightness of the normal light image which is the background image in the superimposed image in the fluorescence observation mode, a user such as a doctor feels strange. In the first mode (fluorescence observation mode), reduction of the first light emission period decreases the brightness of the normal light image, which is the background image in the superimposed image, leading to an occurrence of the above-described phenomenon.

To handle this, modification 2-9 adopts a configuration in which the brightness of the image based on the pixel signal output from the first image sensor 531 in the first mode (fluorescence observation mode) is adjusted to be closer to the brightness of the image in the second mode (normal light observation mode). Modification 2-9 also adopts a configuration in which the digital gain in the first mode (fluorescence observation mode) is set to a value larger than the digital gain in the second mode (normal light observation mode). Specifically, as illustrated in FIG. 17, modification 2-9 has a digital gain controller 944 added to the control unit 94 with respect to the configuration in the second embodiment described above. The digital gain controller 944 corresponds to a brightness adjustment unit according to the present disclosure.

For example, in a case where the first light emission period in the first mode (fluorescence observation mode) is half the period in the second mode (normal light observation mode), the signal value of the pixel signal output from the first image sensor 531 is to be half the signal value in the second mode. As a result, the brightness of the normal light image being the background image in the superimposed image is to be also half the brightness of the normal light image in the second mode (normal light observation mode). To handle this, the digital gain controller 944 sets the digital gain in the first mode (fluorescence observation mode) to a value (for example, double value) larger than the digital gain in the second mode (normal light observation mode). This makes it possible, in the first mode, to bring the brightness of the normal light image being the background image in the superimposed image closer to the brightness of the normal light image in the second mode (normal light observation mode).

As a configuration in which the brightness of the image based on the pixel signal output from the first image sensor 531 in the first mode (fluorescence observation mode) is adjusted to be closer to the brightness of the image in the second mode (normal light observation mode), it is also allowable to adopt an imaging controller 942, not limited to the digital gain controller 944. In this case, the imaging controller 942 corresponds to a brightness adjustment unit according to the present disclosure.

Specifically, the imaging controller 942 sets the analog gain in the first mode (fluorescence observation mode) to a value larger than the analog gain in the second mode (normal light observation mode). Alternatively, the imaging controller 942 sets the exposure time of the second image sensor 532 (opening amount of the electronic shutter) in the first mode (fluorescence observation mode) to a value larger than the exposure time in the second mode (normal light observation mode). This makes it possible, in the first mode, to bring the brightness of the normal light image being the background image in the superimposed image closer to the brightness of the normal light image in the second mode (normal light observation mode).

Even when adopting the configuration of modification 2-9 described above, it is possible to obtain the effects similar to those of the above-described second embodiment.

Note that the normal light image, which is the background image in the superimposed image, may be an image generated based on at least one of the pixel signals output from the red (R) pixel, the green (G) pixel, and the blue (B) pixel in the first image sensor 531. Specifically, the normal light image that is the background image in the superimposed image may be a color image, a pseudo color image using only some colors, or a monochrome image having only luminance information.

(Modification 2-10 of second embodiment)

The configuration of modification 2-7 described above using a plurality of agents may be combined with the configuration of modification 2-9 described above.

Specifically, in modification 2-7 described above, in a case where each image generated in the first and second modes is the superimposed image described in modification 2-9 described above, there may be occurrence of a phenomenon similar to that in modification 2-9 described above. That is, there may be occurrence of a phenomenon in which brightness is different between the normal light image being the background image in the superimposed image in the first mode using the first agent and the normal light image being the background image in the superimposed image in the second mode using the second agent.

Therefore, modification 2-7 described above adopts a configuration, similarly to modification 2-9 described above, in which the brightness of the image based on the pixel signal output from the first image sensor 531 in the first mode is adjusted to be closer to the brightness of the image in the second mode. Furthermore, modification 2-7 described above adopts a configuration, similarly to modification 2-9 described above, in which a parameter in the first mode is set to a value larger than the parameter in the second mode (normal light observation mode). The parameter is at least one of an exposure time of the second image sensor 532 (opening amount of the electronic shutter), an analog gain, and a digital gain. With this configuration, even when using a plurality of agents, the brightness of the normal light image being the background image in the superimposed image in the first mode using the first agent can be brought close to the brightness of the normal light image being the background image in the superimposed image in the second mode using the second agent.

Even when adopting the configuration of modification 2-10 described above, it is possible to obtain the effects similar to those of the above-described second embodiment, modifications 2-7 and 2-9.

(Modification 2-11 of second embodiment)

The configuration of modification 2-8 described above applied to the first to sixth scenes may be combined with the configuration of modification 2-9 described above.

Specifically, in modification 2-8 described above, in a case where each image generated in the first and second modes is the superimposed image described in modification 2-9 described above, there may be occurrence of a phenomenon similar to that in modification 2-9 described above. That is, there may be occurrence of a phenomenon in which brightness is different between a normal light image being a background image in a superimposed image generated in a first surgical scene (first mode) and a normal light image being a background image in a superimposed image generated in a second surgical scene (second mode).

Therefore, modification 2-8 described above adopts a configuration, similarly to modification 2-9 described above, in which the brightness of the image based on the pixel signal output from the first image sensor 531 in the first mode is adjusted to be closer to the brightness of the image in the second mode. Furthermore, modification 2-8 described above adopts a configuration, similarly to modification 2-9 described above, in which a parameter in the first mode is set to a value larger than the parameter in the second mode (normal light observation mode). The parameter is at least one of an exposure time of the second image sensor 532 (opening amount of the electronic shutter), an analog gain, and a digital gain. With this configuration, the brightness of the normal light image being the background image in the superimposed image generated in the first surgical scene (first mode) can be brought close to the brightness of the normal light image being the background image in the superimposed image in the second surgical scene (second mode).

Even when adopting the configuration of modification 2-11 described above, it is possible to obtain the effects similar to those of the above-described second embodiment, modifications 2-7 and 2-9.

(Other embodiments)

While the above is description of the modes for carrying out the present disclosure, the present disclosure should not be limited by only the embodiment described above.

In the first and second embodiments described above, the first and second image sensors 531 and 532 are formed with CMOS, but they are not limited to this and may be formed with a Charge Coupled Device (CCD).

The first and second embodiments have described an exemplary case of the NTSC system and sets 1/60[s] as a reference. However, the reference is not limited to this. In the case of the PAL method, 1/50[s] may be used as a reference, or high-speed imaging operation (for example, 1/240[s]) may be used as a reference. Furthermore, the timings of reading individual fields in the first image sensor 531 and the second image sensor 532 may be the same or may be shifted. For example, in the case of the NTSC system, the timings of reading the respective fields in the first image sensor 531 and the second image sensor 532 may be shifted by 1/120[s]. Furthermore, in the first and second embodiments described above, the first image sensor 531 performs reading at 1/60[s], and the second image sensor 532 performs reading at 1/120[s], but the reading speed of the first and second image sensors 531 and 532 is not limited thereto. That is, the reading speed of the first and second image sensors 531 and 532 may be the same or different. For example, in the case of the NTSC system, both the first and second image sensors 531 and 532 may read at 1/120[s].

In the first mode of the second embodiment described above, the predetermined value (control value in the first control) according to the present disclosure may be changed similarly to the above-described modifications 1-1 to 1-5.

In the first and second embodiments described above, the light source controller 941 adjusts the brightness of the normal light image based on the brightness of a specific area (detection area) in the normal light image. In addition, the light source controller 941 refers to the ratio information stored in the storage unit 97, and executes lighting control of maintaining a state in which the ratio between the light amount of the first light and the light amount of the excitation light is a specific ratio. However, not limited to such lighting control of maintaining the light amount ratio, the light source controller 941 may execute lighting control in which the ratio between the light amount of the first light and the light amount of the excitation light does not become a specific ratio.

For example, the light source controller 941 may adjust the light amount of the excitation light independently of the adjustment of the light amount of the first light in order to adjust the fluorescence image to the reference brightness based on the brightness of a specific area (detection area) in the fluorescence image.

Furthermore, for example, the light source controller 941 may control the operation of the second light source 32 such that the light amount of the excitation light becomes a fixed value (constant value). At this time, the fixed value may be configured to be changeable in accordance with a user operation on the input unit 95.

In the above-described first and second embodiment, the following modifications 3-1 and 3-2 may be adopted.

(Modification 3-1)

The medical observation system according to modification 3-1 is a medical observation system using a device referred to as a video scope (flexible endoscope) including an imaging unit on the distal end side of the insertion unit. Hereinafter, for convenience of description, the medical observation system 1 according to modification 3-1 will be denoted as a medical observation system 1B.

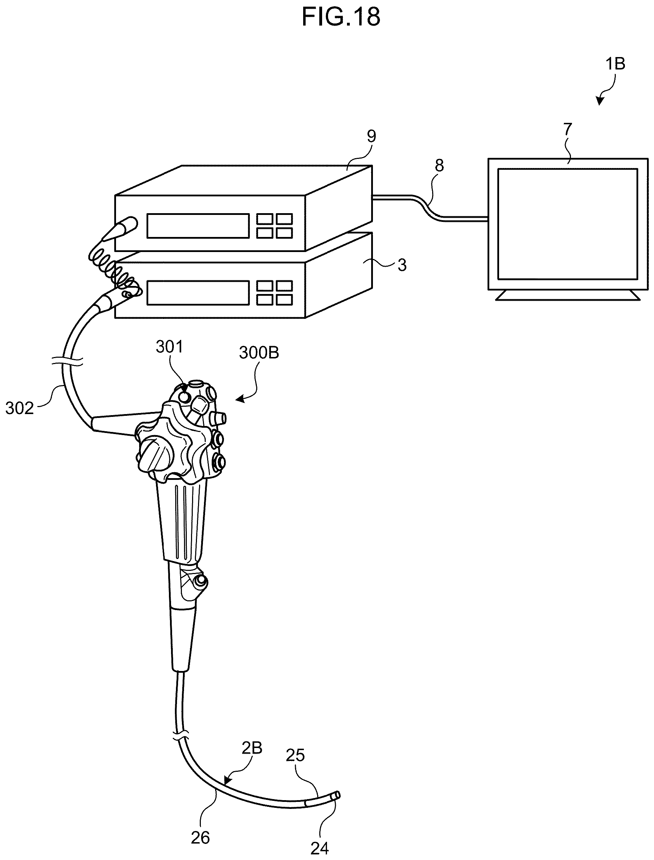

FIG. 18 is a diagram illustrating modification 3-1 of the first and second embodiments.

As illustrated in FIG. 18, the medical observation system 1B includes: an endoscope 300B that captures an in-vivo image of an observed region by inserting an insertion unit 2B into a living body and outputs the captured image; a light source device 3 that emits first light and excitation light from the distal end of the endoscope 300B; a control device 9 that processes the captured image output from the endoscope 300B; and a display device 7 connected to the control device 9 via a second transmission cable 8 and configured to display an image based on a video signal processed by the control device 9.

As illustrated in FIG. 18, the endoscope 300B includes an insertion unit 2B that is a flexible and elongated portion; an operating unit 301 that is connected on a proximal end side of the insertion unit 2B and receives various operations; and a universal cord 302 that extends from the operating unit 301 in a direction different from the extending direction of the insertion unit 2B and incorporates various cables for connecting with the light source device 3 and the control device 9.

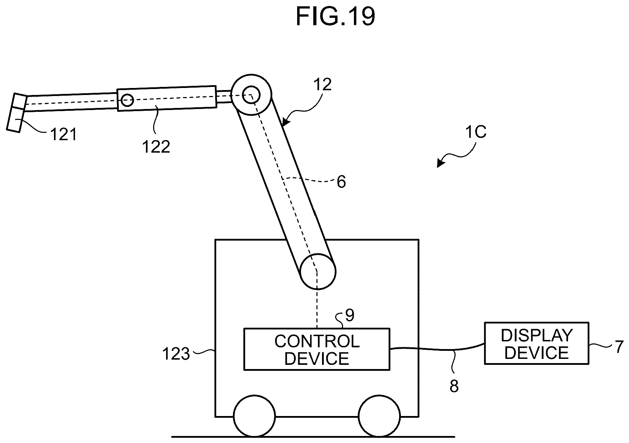

As illustrated in FIG. 19, the insertion unit 2B includes: a distal end portion 24; a bendable portion 25 that is a bendable portion connected to the proximal end side of the distal end portion 24 and is formed with a plurality of bending pieces; and a flexible tube portion 26 that is a flexible and elongated portion connected to the proximal end side of the bendable portion 25.

Although the specific illustration is omitted, the distal end portion 24 incorporates a configuration substantially similar to that of the camera head 5 described above in the first and second embodiments. The captured image captured by the distal end portion 24 (image sensor) is output to the control device 9 via the operating unit 301 and the universal cord 302.

Even when adopting the configuration of modification 3-1 described above, it is possible to obtain the effects similar to those of the above-described first and second embodiments.

(Modification 3-2)

A medical observation system according to modification 3-2 is a medical observation system using a surgical microscope that enlarges and captures a predetermined visual field area inside a subject (inside the living body) or a surface of the subject (surface of the living body) as the observation target. Hereinafter, for convenience of description, the medical observation system 1 according to the third modification will be denoted as a medical observation system 1C.

FIG. 19 is a diagram illustrating modification 3-2 of the first and second embodiments.

As illustrated in FIG. 19, the medical observation system 1C includes: a surgical microscope 12 that captures an image for observing the subject and outputs a captured image; a control device 9 that processes the captured image output from the surgical microscope 12; and a display device 7 that is connected to the control device 9 via a second transmission cable 8 and displays an image based on a video signal processed by the control device 9.

As illustrated in FIG. 19, the surgical microscope 12 includes: a microscope unit 121 that captures an enlarged image of the minute sites of the subject and outputs the captured image; a support 122 connected to the proximal end portion of the microscope unit 121 and that includes an arm that pivotally supports the microscope unit 121; and a base 123 that pivotally holds the proximal end portion of the support 122 and is movable on the floor surface.

As illustrated in FIG. 19, the control device 9 is installed in the base 123. In addition, although not specifically illustrated, the base 123 also includes a light source device 3 that emits the first light and the excitation light from the surgical microscope 12 to the observation target.

The base 123 may also be fixed to the ceiling, wall surface, or the like to support the support 122, instead of being movably provided on the floor surface.

Although the specific illustration is omitted, the microscope unit 121 incorporates a configuration substantially similar to that of the camera head 5 described above in the first and second embodiments. The captured image captured by the imaging on the microscope unit 121 (image sensor) is output to the control device 9 via a first transmission cable 6 wired along the support 122.

Even when adopting the configuration of modification 3-2 described above, it is possible to obtain the effects similar to those of the above-described first and second embodiments.

(Modification 3-3)

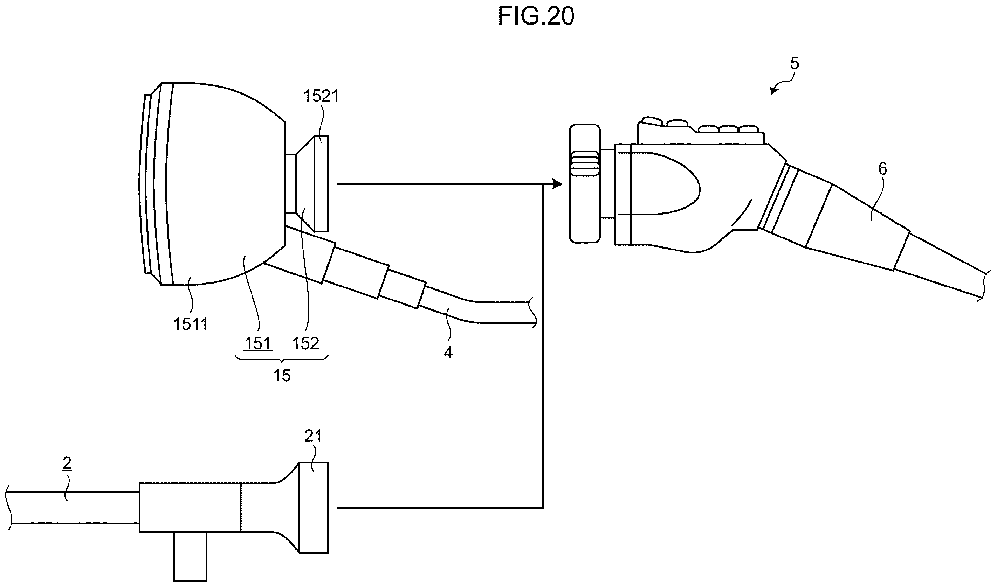

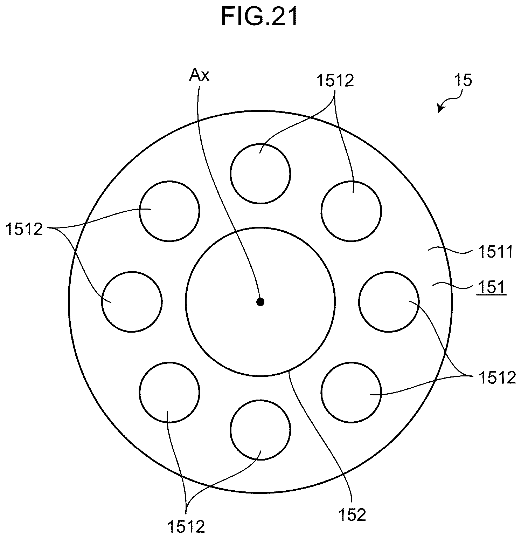

FIGS. 20 and 21 are diagrams illustrating modification 3-3 of the embodiment. Specifically, FIG. 20 is a side view of the ring light 15. FIG. 21 is a diagram of the ring light 15 as viewed from the front side (left side in FIG. 20).

In the fourth modification, in addition to the insertion unit 2 described in the first and second embodiments described above, the ring light 15 illustrated in FIGS. 20 and 21 is detachably connected to the camera head 5. That is, as illustrated in FIG. 20, the insertion unit 2 may be connected to the camera head 5 or the ring light 15 may be connected to the camera head 5 depending on the use state of the user.

Instead of being inserted into the observation target OB like the insertion unit 2, the ring light 15 supplies the first light and the excitation light to the surgical site and captures the second light (subject image) which is return light of the first light and the excitation light from the surgical site. As illustrated in FIGS. 20 and 21, the ring light 15 includes an illumination unit 151 and a subject image capturing unit 152 that captures a subject image.

As illustrated in FIGS. 20 and 21, the illumination unit 151 includes a housing 1511 and a plurality of illumination lenses 1512.

The housing 1511 has an annular shape centered on an optical axis Ax. The light guide 4 has its other end detachably connected to the housing 1511.

As illustrated in FIG. 21, the plurality of illumination lenses 1512 is arranged at predetermined intervals in a circumferential direction centered on the optical axis Ax on the end surface on the front side of the housing 1511. Subsequently, the plurality of illumination lenses 1512 irradiate the surgical site with the first light supplied from the light source device 3 and introduced into the housing 1511 via the light guide 4.

The subject image capturing unit 152 extends along the optical axis Ax. The subject image capturing unit 152 internally includes an optical system formed with one or more lenses and configured to condense the second light (subject image) emitted from the plurality of illumination lenses 1512 and passing through the surgical site. Furthermore, there is provided a connection unit 1521 at an end portion on the proximal end side (right side in FIG. 20) of the subject image capturing unit 152. The connection unit 1521 is designed (shaped) to be compatible with the eyepiece 21 in the insertion unit 2, and is detachably connected to the camera head 5.

Even when adopting the configuration of modification 3-3 described above, it is possible to obtain the effects similar to those of the above-described first and second embodiments.

The following configurations also belong to the technical scope of the present disclosure.

(1) A medical control device including: a light source controller configured to perform: a first control of emitting first light including at least a part of a visible light wavelength band from a light source device; and a second control of emitting excitation light that excites a substance contained in an observation target from the light source device; an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; and a mode switcher configured to switch a mode to a first mode or a second mode, in which the light source controller is configured to set a control value in the first control to a control value of a control threshold or less in the first mode, and set the control value in the first control to a control value larger than the control threshold in the second mode, and the control threshold is a maximum value of the control value in which a signal value based on the observation target fluorescence is larger than a signal value based on unnecessary light imaged by the second image sensor and generated at least due to the first light.

(2) The medical control device according to (1) above, in which the control value in the first control includes at least one of: an emission period of the first light from the light source device; or a current value to be supplied to the light source device to emit the first light.

(3) The medical control device according to (1) or (2) above, further including an operation receiving unit configured to receive a user operation, in which the mode switcher is configured to switch the mode to the first mode or the second mode in accordance with the user operation.

(4) The medical control device according to (1) or (2) above, in which the mode switcher is configured to switch the mode to the first mode or the second mode based on a type of an endoscope connected to the medical control device.

(5) The medical control device according to (1) or (2) above, in which the medical control device has a configuration to which an open field observation device or an endoscope observation device is connectable, and the mode switcher is configured to switch the mode to the second mode when the open field observation device is connected to the medical control device, and switch the mode to the first mode when the endoscope observation device is connected to the medical control device.

(6) The medical control device according to (1) or (2) above, in which the mode switcher is configured to switch the mode to the first mode or the second mode based on a signal value of a pixel signal output from the first image sensor.

(7) The medical control device according to (1) or (2) above, in which the mode switcher is configured to switch the mode to the first mode or the second mode based on a signal value of a pixel signal output from the second image sensor.

(8) The medical control device according to (1) or (2) above, in which the mode switcher is configured to switch the mode to the first mode or the second mode based on a type of a substance contained in the observation target excited by the excitation light.

(9) The medical control device according to any one of (1) to (8) above, in which a control value in the first control is an emission period of the first light from the light source device, and the control threshold is a period equal to or less than a full line exposure period in the first image sensor.

(10) The medical control device according to any one of (1) to (9) above, in which the light source controller is configured to execute the first control in the first mode such that a signal value based on unnecessary light imaged by the second image sensor and generated at least due to the first light becomes smaller than the signal value based on the unnecessary light in the second mode.

(11) The medical control device according to any one of (1) to (10) above, in which the first mode is a fluorescence observation mode of generating a display image based on pixel signals individually output from the first image sensor and the second image sensor, and the second mode is a normal light observation mode of generating the display image based on a pixel signal output from only the first image sensor among the first image sensor and the second image sensor.

(12) The medical control device according to any one of (1) to (10) above, in which the first mode is a mode corresponding to a first agent that emits the observation target fluorescence by irradiation with the excitation light, and the second mode is a mode corresponding to a second agent that is an agent different from the first agent and emits the observation target fluorescence by irradiation with the excitation light.

(13) The medical control device according to (12) above, in which intensity of the observation target fluorescence based on the first agent is lower than intensity of the observation target fluorescence based on the second agent.

(14) The medical control device according to any one of (1) to (10) above, in which the first mode is a mode corresponding to a first surgical scene, and the second mode is a mode corresponding to a second surgical scene different from the first surgical scene.

(15) The medical control device according to (12) above4, in which intensity of the observation target fluorescence in the first surgical scene is lower than intensity of the observation target fluorescence in the second surgical scene.

(16) A medical control device including: a light source controller configured to perform: a first control of emitting first light including at least a part of a visible light wavelength band from a light source device; and a second control of emitting excitation light that excites a substance contained in an observation target from the light source device; an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; a mode switcher configured to switch a mode to a first mode or a second mode; and a brightness controller configured to control brightness of an image based on a pixel signal output from the first image sensor, in which the light source controller is configured to set a control value in the first control to a control value of a control threshold or less in the first mode, and set the control value in the first control to a control value larger than the control threshold in the second mode, and the brightness controller is configured to control the brightness such that the brightness of the image based on the pixel signal output from the first image sensor in the first mode is adjusted to be closer to the brightness of the image in the second mode.

(17) A medical control device including: a light source controller configured to perform: a first control of emitting first light including at least a part of a visible light wavelength band from a light source device; and a second control of emitting excitation light that excites a substance contained in an observation target from the light source device; an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; a mode switcher configured to switch to a first mode or a second mode; and a brightness controller configured to adjust brightness of an image based on a pixel signal output from the first image sensor, in which the light source controller is configured to set a control value in the first control to a control value of a control threshold or less in the first mode, and set the control value in the first control to a control value larger than the control threshold in the second mode, the brightness controller is configured to set a parameter in the first mode to a larger value than the parameter in the second mode, and the parameter is at least one of: an exposure time of the first image sensor; an analog gain to adjust the brightness of the image based on the pixel signal output from the first image sensor; or a digital gain to adjust the brightness of the image based on the pixel signal output from the first image sensor.

(18) A medical observation system including: a light source device configured to emit each light of first light including at least a part of a visible light wavelength band and excitation light that excites a substance contained in an observation target; an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and a medical control device configured to control individual operations of the light source device and the imaging device, in which the medical control device includes: a light source controller configured to execute: a first control of emitting the first light from the light source device; and a second control of emitting the excitation light from the light source device; an imaging controller configured to control operations of the imaging device; and a mode switcher is configured to switch a mode to a first mode or a second mode, the light source controller is configured to set a control value in the first control to a control value of a control threshold or less in the first mode, and set the control value in the first control to a control value larger than the control threshold in the second mode, and the control threshold is a maximum value of the control value in which a signal value based on the observation target fluorescence is larger than a signal value based on unnecessary light imaged by the second image sensor and generated at least due to the first light.

(19) The medical observation system according to (18) above, in which the first light is broadband light or narrowband light.

(20) The medical observation system according to (18) or (19) above, in which the light source device includes a first light source configured to emit the first light, and the first light source is provided as one light source or a plurality of light sources.

(21) The medical observation system according to any one of (18) to (20) above, in which the light source device includes a first light source configured to emit the first light, and the first light source includes an LED or a semiconductor laser.

(22) The medical observation system according to any one of (18) to (21) above, in which the excitation light is narrowband light.

(23) The medical observation system according to any one of (18) to (22) above, in which the light source device includes a second light source configured to emit the excitation light, and the second light source is provided as one light source or a plurality of light sources.

(24) The medical observation system according to any one of (18) to (23) above, in which the light source device includes a second light source configured to emit the excitation light, and the second light source includes an LED or a semiconductor laser.

(25) A medical observation system including: a light source device configured to emit each light of first light including at least a part of a visible light wavelength band and excitation light that excites a substance contained in an observation target; an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and a medical control device configured to control individual operations of the light source device and the imaging device, in which the medical control device includes: a light source controller configured to execute: a first control of emitting the first light from the light source device; and a second control of emitting the excitation light from the light source device; an imaging controller configured to control operations of the imaging device; a mode switcher configured to switch a mode to a first mode or a second mode; and a brightness controller configured to control brightness of an image based on a pixel signal output from the first image sensor, the light source controller is configured to set a control value in the first control in the first mode to a control value of a control threshold or less, and set the control value in the first control in the second mode to a control value larger than the control threshold, and the brightness controller is configured to control the brightness such that the brightness of the image based on the pixel signal output from the first image sensor in the first mode is adjusted to be closer to the brightness of the image in the second mode.

(26) A medical observation system including: a light source device configured to emit each light of first light including at least a part of a visible light wavelength band and excitation light that excites a substance contained in an observation target; an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and a medical control device configured to control individual operations of the light source device and the imaging device, in which the medical control device includes: a light source controller configured to execute: a first control of emitting the first light from the light source device; and a second control of emitting the excitation light from the light source device; an imaging controller configured to control operations of the imaging device; a mode switcher configured to switch a mode to a first mode or a second mode; and a brightness controller that controls brightness of an image based on a pixel signal output from the first image sensor, the light source controller is configured to set a control value in the first control in the first mode to a control value of a control threshold or less, and set the control value in the first control in the second mode to a control value larger than the control threshold, and the brightness controller is configured to set a parameter in the first mode to a value larger than the parameter in the second mode, and the parameter is at least one of: an exposure time of the first image sensor; an analog gain to adjust the brightness of the image based on the pixel signal output from the first image sensor; or a digital gain to adjust the brightness of the image based on the pixel signal output from the first image sensor.

Reference Signs List

| 1, 1A to 1C | MEDICAL OBSERVATION SYSTEM |
| --- | --- |
| 2, 2B | INSERTION UNIT |
| 3 | LIGHT SOURCE DEVICE |
| 4 | LIGHT GUIDE |
| 5 | CAMERA HEAD |
| 6 | FIRST TRANSMISSION CABLE |
| 7 | DISPLAY DEVICE |
| 8 | SECOND TRANSMISSION CABLE |
| 9 | CONTROL DEVICE |
| 10 | THIRD TRANSMISSION CABLE |
| 12 | SURGICAL MICROSCOPE |
| 15 | RING LIGHT |
| 21 | EYEPIECE |
| 22 | EXCITATION LIGHT CUT FILTER |
| 23 | MEMORY |
| 24 | DISTAL END PORTION |
| 25 | BENDABLE PORTION |
| 26 | FLEXIBLE TUBE PORTION |
| 31 | FIRST LIGHT SOURCE |
| 32 | SECOND LIGHT SOURCE |
| 33 | THIRD LIGHT SOURCE |
| 34 | FOURTH LIGHT SOURCE |
| 51 | LENS UNIT |
| 52 | PRISM |
| 53 | IMAGING UNIT |

| 54 | COMMUNICATION UNIT |
|---|---|
| 91 | COMMUNICATION UNIT |
| 92 | IMAGE MEMORY |
| 93 | PROCESSING MODULE |
| 94 | CONTROL UNIT |
| 95 | INPUT UNIT |
| 96 | OUTPUT UNIT |
| 97 | STORAGE UNIT |
| 100 | ENDOSCOPE OBSERVATION DEVICE |
| 101 | MEMORY |
| 121 | MICROSCOPE UNIT |
| 122 | SUPPORT |
| 123 | BASE |
| 151 | ILLUMINATION UNIT |
| 152 | SUBJECT IMAGE CAPTURING UNIT |
| 200 | OPEN FIELD OBSERVATION DEVICE |
| 201 | MEMORY |
| 300B | ENDOSCOPE |
| 301 | OPERATING UNIT |
| 302 | UNIVERSAL CORD |
| 531 | FIRST IMAGE SENSOR |
| 532 | SECOND IMAGE SENSOR |
| 533 | SIGNAL PROCESSING UNIT |
| 931 | MEMORY CONTROLLER |
| 932 | FIRST IMAGE PROCESSING UNIT |
| 933 | SECOND IMAGE PROCESSING UNIT |
| 934 | DISPLAY CONTROLLER |
| 941 | LIGHT SOURCE CONTROLLER |
| 942 | IMAGING CONTROLLER |

| 943 | MODE SWITCHER |
| 944 | DIGITAL GAIN CONTROLLER |
| 1511 | HOUSING |
| 1512 | ILLUMINATION LENS |
| 1521 | CONNECTION UNIT |
| Ar1, Ar2 | AREA |
| Ax | OPTICAL AXIS |
| CN1, CN2 | CONNECTOR |
| F1 | FIRST SUBJECT |
| F2 | SECOND SUBJECT |
| L1 | UNNECESSARY LIGHT |
| L2 | OBSERVATION TARGET FLUORESCENCE |
| OB | OBSERVATION TARGET |
| P0 | OBSERVATION OPTICAL PATH |
| P1 | FIRST OPTICAL PATH |
| P2 | SECOND OPTICAL PATH |
| TE | FULL LINE EXPOSURE PERIOD |

**Claims**

1.  A medical control device comprising:

    a light source controller configured to perform: a first control of emitting first light including at least a part of a visible light wavelength band from a light source device; and a second control of emitting excitation light that excites a substance contained in an observation target from the light source device;
    an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light; and
    a mode switcher configured to switch a mode to a first mode or a second mode,
    wherein the light source controller is configured to

    set a control value in the first control to a control value of a control threshold or less in the first mode, and
    set the control value in the first control to a control value larger than the control threshold in the second mode,
    and

    the control threshold is a maximum value of the control value in which a signal value based on the observation target fluorescence is larger than a signal value based on unnecessary light imaged by the second image sensor and generated at least due to the first light.

2.  The medical control device according to claim 1, wherein the control value in the first control includes at least one of: an emission period of the first light from the light source device; or a current value to be supplied to the light source device to emit the first light.

3. The medical control device according to claim 1, further comprising an operation receiving unit configured to receive a user operation,
wherein the mode switcher is configured to switch the mode to the first mode or the second mode in accordance with the user operation.

4. The medical control device according to claim 1, wherein the mode switcher is configured to switch the mode to the first mode or the second mode based on a type of an endoscope connected to the medical control device.

5. The medical control device according to claim 1, wherein

the medical control device has a configuration to which an open field observation device or an endoscope observation device is connectable, and
the mode switcher is configured to

switch the mode to the second mode when the open field observation device is connected to the medical control device, and
switch the mode to the first mode when the endoscope observation device is connected to the medical control device.

6. The medical control device according to claim 1, wherein the mode switcher is configured to switch the mode to the first mode or the second mode based on a signal value of a pixel signal output from the first image sensor.

7. The medical control device according to claim 1, wherein the mode switcher is configured to switch the mode to the first mode or the second mode based on a signal value of a pixel signal output from the second image sensor.

8. The medical control device according to claim 1, wherein the mode switcher is configured to switch the mode to the first mode or the second mode based on a type of a substance contained in the observation target excited by the excitation light.

9. The medical control device according to claim 1, wherein a control value in the first control is an emission period of the first light from the light source device, and
the control threshold is a period equal to or less than a full line exposure period in the first image sensor.

10. The medical control device according to claim 1, wherein the light source controller is configured to execute the first control in the first mode such that a signal value based on unnecessary light imaged by the second image sensor and generated at least due to the first light becomes smaller than the signal value based on the unnecessary light in the second mode.

11. The medical control device according to claim 1, wherein the first mode is a fluorescence observation mode of generating a display image based on pixel signals individually output from the first image sensor and the second image sensor, and
the second mode is a normal light observation mode of generating the display image based on a pixel signal output from only the first image sensor among the first image sensor and the second image sensor.

12. The medical control device according to claim 1, wherein the first mode is a mode corresponding to a first agent that emits the observation target fluorescence by irradiation with the excitation light, and
the second mode is a mode corresponding to a second agent that is an agent different from the first agent and emits the observation target fluorescence by irradiation with the excitation light.

13. The medical control device according to claim 12, wherein intensity of the observation target fluorescence based on the first agent is lower than intensity of the observation target fluorescence based on the second agent.

14. The medical control device according to claim 1, wherein the first mode is a mode corresponding to a first surgical scene, and
the second mode is a mode corresponding to a second surgical scene different from the first surgical scene.

15. The medical control device according to claim 14, wherein intensity of the observation target fluorescence in the first surgical scene is lower than intensity of the observation target fluorescence in the second surgical scene.

16. A medical control device comprising:

a light source controller configured to perform: a first control of emitting first light including at least a part of a visible light wavelength band from a light source device; and a second control of emitting excitation light that excites a substance contained in an observation target from the light source device;

an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light;

a mode switcher configured to switch a mode to a first mode or a second mode; and

a brightness controller configured to control brightness of an image based on a pixel signal output from the first image sensor,

wherein the light source controller is configured to

set a control value in the first control to a control value of a control threshold or less in the first mode, and set the control value in the first control to a control value larger than the control threshold in the second mode, and

the brightness controller is configured to control the brightness such that the brightness of the image based on the pixel signal output from the first image sensor in the first mode is adjusted to be closer to the brightness of the image in the second mode.

17. A medical control device comprising:

a light source controller configured to perform: a first control of emitting first light including at least a part of a visible light wavelength band from a light source device; and a second control of emitting excitation light that excites a substance contained in an observation target from the light source device;

an imaging controller configured to control operations of: a first image sensor configured to image light including at least a part of a visible light wavelength band among second light being return light of the first light and the excitation light from the observation target; and a second image sensor configured to image light including at least a part of a wavelength band of observation target fluorescence emitted from the observation target by irradiation with the excitation light among the second light;

a mode switcher configured to switch to a first mode or a second mode; and

a brightness controller configured to adjust brightness of an image based on a pixel signal output from the first image sensor,

wherein the light source controller is configured to

set a control value in the first control to a control value of a control threshold or less in the first mode, and set the control value in the first control to a control value larger than the control threshold in the second mode,

the brightness controller is configured to set a parameter in the first mode to a larger value than the parameter in the second mode, and

the parameter is at least one of: an exposure time of the first image sensor; an analog gain to adjust the brightness of the image based on the pixel signal output from the first image sensor; or a digital gain to adjust the brightness of the image based on the pixel signal output from the first image sensor.

18. A medical observation system comprising:

a light source device configured to emit each light of first light including at least a part of a visible light wavelength band and excitation light that excites a substance contained in an observation target;

an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and
a medical control device configured to control individual operations of the light source device and the imaging device,
wherein the medical control device includes:

a light source controller configured to execute: a first control of emitting the first light from the light source device; and a second control of emitting the excitation light from the light source device;
an imaging controller configured to control operations of the imaging device; and

a mode switcher is configured to switch a mode to a first mode or a second mode,
the light source controller is configured to

set a control value in the first control to a control value of a control threshold or less in the first mode, and
set the control value in the first control to a control value larger than the control threshold in the second mode, and

the control threshold is a maximum value of the control value in which a signal value based on the observation target fluorescence is larger than a signal value based on unnecessary light imaged by the second image sensor and generated at least due to the first light.

19. The medical observation system according to claim 18, wherein the first light is broadband light or narrowband light.

20. The medical observation system according to claim 18, wherein the light source device includes a first light source configured to emit the first light, and
the first light source is provided as one light source or a plurality of light sources.

21. The medical observation system according to claim 18, wherein the light source device includes a first light source configured to emit the first light, and
the first light source includes an LED or a semiconductor laser.

22. The medical observation system according to claim 18, wherein the excitation light is narrowband light.

23. The medical observation system according to claim 18, wherein the light source device includes a second light source configured to emit the excitation light, and
the second light source is provided as one light source or a plurality of light sources.

24. The medical observation system according to claim 18, wherein the light source device includes a second light source configured to emit the excitation light, and
the second light source includes an LED or a semiconductor laser.

25. A medical observation system comprising:

a light source device configured to emit each light of first light including at least a part of a visible light wavelength band and excitation light that excites a substance contained in an observation target;
an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and
a medical control device configured to control individual operations of the light source device and the imaging device,
wherein the medical control device includes:

a light source controller configured to execute: a first control of emitting the first light from the light source device; and a second control of emitting the excitation light from the light source device;
an imaging controller configured to control operations of the imaging device;

a mode switcher configured to switch a mode to a first mode or a second mode; and
a brightness controller configured to control brightness of an image based on a pixel signal output from the first image sensor,

the light source controller is configured to

set a control value in the first control in the first mode to a control value of a control threshold or less, and set the control value in the first control in the second mode to a control value larger than the control threshold, and

the brightness controller is configured to control the brightness such that the brightness of the image based on the pixel signal output from the first image sensor in the first mode is adjusted to be closer to the brightness of the image in the second mode.

26. A medical observation system comprising:

a light source device configured to emit each light of first light including at least a part of a visible light wavelength band and excitation light that excites a substance contained in an observation target;
an imaging device including a first image sensor and a second image sensor, the first image sensor being configured to image light including at least a part of the visible light wavelength band among second light being return light of the first light and the excitation light from the observation target, the second image sensor being configured to image light including at least a part of a wavelength band of observation target fluorescence emitted from the observation target by irradiation with the excitation light among the return light of the visible light and the excitation light; and
a medical control device configured to control individual operations of the light source device and the imaging device,
wherein the medical control device includes:

a light source controller configured to execute: a first control of emitting the first light from the light source device; and a second control of emitting the excitation light from the light source device;
an imaging controller configured to control operations of the imaging device;
a mode switcher configured to switch a mode to a first mode or a second mode; and
a brightness controller that controls brightness of an image based on a pixel signal output from the first image sensor,

the light source controller is configured to

set a control value in the first control in the first mode to a control value of a control threshold or less, and set the control value in the first control in the second mode to a control value larger than the control threshold, and

the brightness controller is configured to set a parameter in the first mode to a value larger than the parameter in the second mode, and
the parameter is at least one of: an exposure time of the first image sensor; an analog gain to adjust the brightness of the image based on the pixel signal output from the first image sensor; or a digital gain to adjust the brightness of the image based on the pixel signal output from the first image sensor.

# FIG.1

# FIG.2

# FIG.3

# FIG.4

# FIG.5

# FIG.6

# FIG.7

EP 4 736 737 A1

# FIG.8

EP 4 736 737 A1

FIG.9

FIG.10

# FIG.11

# FIG.12

CAMERA HEAD — 5

IMAGING UNIT — 53

- LENS UNIT — 51
- PRISM — 52
- FIRST IMAGE SENSOR — 531
- SIGNAL PROCESSING UNIT — 533
- SECOND IMAGE SENSOR — 532
- COMMUNICATION UNIT — 54

6

CONTROL DEVICE — 9

COMMUNICATION UNIT — 91

PROCESSING MODULE — 93

- MEMORY CONTROLLER — 931
- FIRST IMAGE PROCESSING UNIT — 932
- SECOND IMAGE PROCESSING UNIT — 933
- DISPLAY CONTROLLER — 934

IMAGE MEMORY — 92

CONTROL UNIT — 94

- LIGHT SOURCE CONTROLLER — 941
- IMAGING CONTROLLER — 942
- MODE SWITCHER — 943

- INPUT UNIT — 95
- OUTPUT UNIT — 96
- STORAGE UNIT — 97

DISPLAY DEVICE — 7

8

1A

LIGHT SOURCE DEVICE — 3

- FIRST LIGHT SOURCE — 31
- SECOND LIGHT SOURCE — 32

# FIG.13

```
                ⌇2                                    ⌇9
┌──────────────────────────┐      ┌──────────────────────────────┐
│ INSERTION UNIT           │      │                              │
│                          │      │                              │
│              ⌇23         │      │                              │
│ ┌──────────────────────┐ │      │       CONTROL DEVICE         │
│ │     MEMORY           │─┼──────┼──►                           │
│ └──────────────────────┘ │      │                              │
│                          │      │                              │
└──────────────────────────┘      └──────────────────────────────┘
```

# FIG.14

```
                ⌇100                                  ⌇9
┌──────────────────────────┐      ┌──────────────────────────────┐
│ ENDOSCOPE                │      │                              │
│ OBSERVATION DEVICE       │      │                              │
│              ⌇101        │      │                              │
│ ┌──────────────────────┐ │      │       CONTROL DEVICE         │
│ │     MEMORY           │─┼──────┼──►                           │
│ └──────────────────────┘ │      │                              │
│                          │      │                              │
└──────────────────────────┘      └──────────────────────────────┘

                ⌇200
┌──────────────────────────┐
│ OPEN FIELD OBSERVATION   │
│ DEVICE                   │
│              ⌇201        │
│ ┌──────────────────────┐ │
│ │     MEMORY           │─┼──────┘
│ └──────────────────────┘ │
│                          │
└──────────────────────────┘
```

FIG.15

1A

CONTROL DEVICE 9

DISPLAY DEVICE 7

8

COMMUNICATION UNIT 91

PROCESSING MODULE 93
- MEMORY CONTROLLER 931
- FIRST IMAGE PROCESSING UNIT 932
- SECOND IMAGE PROCESSING UNIT 933
- DISPLAY CONTROLLER 934

IMAGE MEMORY 92

CONTROL UNIT 94
- LIGHT SOURCE CONTROLLER 941
- IMAGING CONTROLLER 942
- MODE SWITCHER 943

INPUT UNIT 95
OUTPUT UNIT 96
STORAGE UNIT 97

LIGHT SOURCE DEVICE 3
- FIRST LIGHT SOURCE 31
- SECOND LIGHT SOURCE 32
- THIRD LIGHT SOURCE 33

CAMERA HEAD 5
- LENS UNIT 51
- PRISM 52
- IMAGING UNIT 53
  - FIRST IMAGE SENSOR 531
  - SECOND IMAGE SENSOR 532
  - SIGNAL PROCESSING UNIT 533
- COMMUNICATION UNIT 54

6

FIG.16

1A

**DISPLAY DEVICE** 7

**CONTROL DEVICE** 9

8

**IMAGE MEMORY** 92

**COMMUNICATION UNIT** 91

**PROCESSING MODULE** 93
- **MEMORY CONTROLLER** 931
- **FIRST IMAGE PROCESSING UNIT** 932
- **SECOND IMAGE PROCESSING UNIT** 933
- **DISPLAY CONTROLLER** 934

**CONTROL UNIT** 94
- **LIGHT SOURCE CONTROLLER** 941
- **IMAGING CONTROLLER** 942
- **MODE SWITCHER** 943

**INPUT UNIT** 95
**OUTPUT UNIT** 96
**STORAGE UNIT** 97

**LIGHT SOURCE DEVICE** 3
- **FIRST LIGHT SOURCE** 31
- **SECOND LIGHT SOURCE** 32
- **THIRD LIGHT SOURCE** 33
- **FOURTH LIGHT SOURCE** 34

6

**CAMERA HEAD** 5

**IMAGING UNIT** 53
- **COMMUNICATION UNIT** 54
- **SIGNAL PROCESSING UNIT** 533
- **FIRST IMAGE SENSOR** 531
- **SECOND IMAGE SENSOR** 532

**PRISM** 52
**LENS UNIT** 51

# FIG.17

EP 4 736 737 A1

# FIG.18

# FIG.19

# FIG.20

EP 4 736 737 A1

# FIG.21

## INTERNATIONAL SEARCH REPORT

| International application No. |
| --- |
| **PCT/JP2024/023262** |

### A. CLASSIFICATION OF SUBJECT MATTER

***A61B 1/06***(2006.01)i; ***A61B 1/00***(2006.01)i
FI:   A61B1/06 613; A61B1/00 511

According to International Patent Classification (IPC) or to both national classification and IPC

### B. FIELDS SEARCHED

Minimum documentation searched (classification system followed by classification symbols)

A61B1/06; A61B1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| X | JP 2007-90044 A (OLYMPUS CORPORATION) 12 April 2007 (2007-04-12) paragraphs [0031]-[0032], [0056]-[0086], [0129]-[0189], fig. 11-12, 27-35 | 1, 3, 10-11, 16-22, 25-26 |
| A | paragraphs [0031]-[0032], [0056]-[0086], [0129]-[0189], fig. 11-12, 27-35 | 2, 4-9, 12-15, 23-24 |
| A | JP 2017-524924 A (UNIV SINGAPORE) 31 August 2017 (2017-08-31) entire text, all drawings | 1-26 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **06 August 2024** | **20 August 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/023262**

| Patent document cited in search report | | | Publication date (day/month/year) | Patent family member(s) | | | Publication date (day/month/year) |
|---|---|---|---|---|---|---|---|
| JP | 2007-90044 | A | 12 April 2007 | US | 2007/0046778 | A1 | |
| | | | | paragraphs [0109]-[0110], [0134]-[0164], [0207]-[0267], fig. 11-12, 27-35 | | | |
| | | | | EP | 1759628 | A1 | |
| JP | 2017-524924 | A | 31 August 2017 | US | 2017/0138860 | A1 | |
| | | | | entire text, all drawings | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021132695 A
- JP 6160731 A